# EUROPEAN PATENT SPECIFICATION

(11) **EP 1 984 382 B1**
(45) Date of publication and mention of the grant of the patent: **15.08.2012**
(21) Application number: 07711406.4
(22) Date of filing: 29.01.2007
(51) Int. Cl.: C07H 19/06

(54) **LNA MODIFIED PHOSPHOROTHIOLATED OLIGONUCLEOTIDES**
LNA-MODIFIZIERTE PHOSPHOROTHIOLIERTE OLIGONUKLEOTIDE
OLIGONUCLÉOTIDES PHOSPHORÉS THIOLÉS MODIFIÉS PAR DES ACIDES NUCLÉIQUES VERROUILLÉS

(30) Priority: 27.01.2006 US 762920 P
(43) Date of publication of application: 29.10.2008
(73) Proprietor: Santaris Pharma A/S, 2970 Hørsholm (DK)
(72) Inventor: ELMEN, Joacim, 120 67 Stockholm (SE); HANSEN, Henrik, Frydenlund, DK-2610 Rødovre (DK); øRUM, Henrik, DK-3500 Vaerløse (DK); KOCH, Troels, DK-3200 Copenhagen (DK)
(74) Representative: Turner, Mark Frederic Paris
(86) International application number: PCT/EP2007/000741
(87) International publication number: WO 2007/085485

(56) References cited:
- WO-A-2006/050734
- WO-A2-03/070918
- KUMAR R ET AL: "The first Analogues of LNA (Locked Nucleic Acids) : Phosphorothioate-LNA and 2'-Thio-LNA" BIOORGANIC & MEDICINAL CHEMISTRY LETTERS, OXFORD, GB, no. 8, 1998, pages 2219-2222, XP002094302 ISSN: 0960-894X
- FRIEDEN M ET AL: "Expanding the design horizon of antisense oligonucleotides with alpha-L-LNA" NUCLEIC ACIDS RESEARCH, OXFORD UNIVERSITY PRESS, SURREY, GB, vol. 31, no. 21, 1 November 2003 (2003-11-01), pages 6365-6372, XP002281376 ISSN: 0305-1048

## Description

### FIELD OF THE INVENTION

The current invention provides oligonucleotides which comprise a dinucleotide consisting of a 5' locked nucleic acid (LNA), a phosphorothioate internucloside linkage bond to a 3' RNA or RNA analogue. The dinucleotide reduces the strength of hybridization of the oligonucleotide to a complementary nucleic acid target. The modification can be used to modulate hybridisation properties in both single stranded oligonucleotides and in double stranded siRNA complexes, and microRNA mimics, particularly in oligocnucleotides where the use of LNA results in excessively strong hybridisation properties.

### BACKGROUND OF THE INVENTION

LNA has an extraordinary ability to protect oligonucleotides from nuclease degradation and at the same time increase affinity for its complementary target. These are usually highly desirable properties for nucleic acid based gene-silencing techniques.

Gene-silencing mechanisms, such as RNA interference (RNAi), require RNA like structures for function. It has been shown that the RNAI cellular machinery recognises LNA, and as such LNA based oligonucleotides may be used to down-regulate target molecules via RNAi or similar mechanisms.

The effector in RNAi is small interfering RNAs (siRNA), short (21-23 nt) double stranded RNA oligonucleotides. The gene-silencing potential of siRNA has been proved *in vitro.* However, in a therapeutic setting these molecules have not yet proven as useful as traditional single stranded antisense oligonucleotides. One reason for this is commonly thought to be uptake, the siRNA being double stranded (i.e. dsRNAi), has at least twice the molecular weight of standard antisense oligonucleotides. The increased size causes the cost of synthesis to be higher. Hence, a single stranded oligonucleotide capable of utilizing an RNAi mechanism would be ideal (ssRNAi).

It has been shown that a single stranded RNA oligonucleotide can perform RNAi, however with very low efficiency. This is postulated to be due to the extremely unstable nature of singe stranded RNA and then the inability for the intact RNA strand to reach and be incorporated into the effector protein complex (RNA induced silencing complex, RISC).

LNA can be used to enhance the nuclease resistance. However, due to the extremely unstable nature of RNA, a fairly high load of LNA is required for nuclease protection. As mentioned a high load of LNA also gives a high affinity (measured as melting temperature, Tₘ), which in turn can reduce the RNAi gene-silencing kinetics, thought to be due to hindering the separation of the two strands (dsRNAI), and/or target strand release (such as in ssRNAi). (possibly reducing unwinding kinetics, in case of double stranded RNA and target release in case of both single and double stranded RNA).

The use of LNA in single stranded antisense oligonucleotides is highly beneficial, providing vastiy improved hybridisation kinetics, enhanced nuclease resistance. However, the number of LNA units which can be used may, in some circumstances be limited, as the affinity to target molecules may become excessive which may then result in a sub-optimal pharmacological profile.

The present invention provides novel combinations of LNA and phosphorythlolated diester bonds that can be used to modulate excessive affinity while maintaining nuclease resistance, creating an optimal, cost effective, single stranded oligonucleotide for RNAi and similar mechanisms as well as traditional antisense therapeutics.

In the case of double stranded siRNA the combination can be used to create nuclease resistant siLNA (LNA modified siRNA) species with optimal Tₘ for maximal gene-silencing.

Phosphorothiolation is beneficial for the pharmacodynamic properties but contribute little to nuclease resistance and nothing to affinity. For RNAi gene-silencing LNA can be combined with phosphorothioates in certain ways to both increase and decrease affinity.

### BRIER DESCRIPTION OF THE INVENTION

The invention provides for a double stranded oligonucleotide according to claim 1.

The invention originates from a most surprising observation that the linkage 3' to LNA in LNA/RNA oligonucleotide modulates Tₘ. Specifically, in a dinucleotide of sequence 5' LNA-PS-XNA 3', the phospborythiolation (P=S) in the 3' position to LNA decreases Tₘ, whereas a phosphodiester 3' to the LNA increases Tm (see Table 1). The use of a dinucleotide of sequence 5' LNA-PS-XNA 3' may decrease the Tₘ up to about 10°C, as compared to an equivalent oligonucleotide where the LNA-XNA linkage is a phosphodiester (P=O) linkage.

**TABLE 1 LNA MONOMER**

| Backbone environment (complement RNA P=O or P=S) | Terminal LNA, ΔTm | Internal LNA, ΔTm |
|---|---|---|
| RNA P=O | + 2-3°C | + 3-4°C |
| RNA P=S | - 0-2°C | - 5-10°C |

In one aspect the mixed sequence oligonucleotide comprises 15-25 nucleotides and having between 4-24 RNA units, between 1-8 LNA units and at least one phosphorothioate linkage.

In another aspect the mixed sequence oligonucleotide comprising between 17-22 nucleotides and having between 11-20 RNA units, between 2-6 LNA units and at least one phosphorothloate linkage.

In one embodiment of the invention, the mixed sequence oligonucleotide is a microRNA sequence or a microRNA mimic. The miRBase (http://microma.sancer.ac.uk/). provides numerous identified miRNAs. Suitably, the oligonucleotide according to the invention may be a miRNA mimic, which may, for example be used to increase the cellular content of a specific microRNA sequence, such as when the microRNA is missing or concentration is diminished. Such siRNA mimics may therefore be used in diseases which are characterised by reduced levels of or absence of specific miRNAs.

In one embodiment, the double stranded oligonucleotide of the invention may be characterised in that the melting temperature (Tₘ) of the duplex is no greater than +/- 10 °C *(i.e.* within a range of + 10 to -10°C) when compared to the Tₘ of a corresponding double stranded oligonucleotide duplex consisting solely of RNA.

In one embodiment, the double stranded oligonucleotide of the invention may be characterised in that the melting temperature (Tₘ) of the duplex is no greater than +/- 7 °C *(i.e.* within a range of + 7 to - 7°C) when compared to the Tₘ of a corresponding double stranded oligonucleotide duplex consisting solely of RNA.

In one embodiment, the double stranded oligonucleotide can have a Tₘ which is no greater than +/- 1°C, +/- 2°C, +/- 3°C, +/- 4°C, +/- 5°C**,** +/- 6°C when compared to the Tₘ of a corresponding double stranded oligonucleotide duplex consisting solely of RNA.

In one embodiment, the double stranded oligonucleotide can have a Tₘ which is no greater than + 1°C, + 2°C, + 3°C, + 4°C, + 5°C, + 6°C when compared to the Tₘ of a corresponding double stranded oligonucleotide duplex consisting solely of RNA.

In one embodiment, the mixed sequence oligonucleotide can have a Tₘ in a duplex with a complementary RNA molecule (phosphate linkages), which is no greater than - 1°C, -2°C, -3°C, -4°C, -5°C or -6°C (i.e. does not have a Tₘ which is lower than -6°C) when compared to the Tₘ of a duplex between a corresponding mixed sequence oligonucleotide consisting soiely of RNA and the complementary RNA molecule.

In one embodiment, the mixed sequence of oligonucleotide can have a Tₘ in a duplex with a complementary RNA molecule which is no greater than +/- 7 °C (*i.e*. within a range of + 7 to -7°C) when compared to the Tₘ of a duplex between a corresponding mixed sequence oligonucleotide consisting solely of RNA and the complementary RNA molecule.

In one embodiment, the mixed sequence oligonucleotide can have a Tₘ in a duplex with a complementary RNA molecule which is no greater than +/- 1°C, +/- 2°C, +/- 3°C, +/-4°C, +/- 5°C, +/- 6°C when compared to the Tₘ of a duplex between a corresponding mixed sequence oligonucleotide consisting solely of RNA and the complementary RNA molecule.

In one embodiment, the mixed sequence oligonucleotide can have a Tₘ in a duplex with a complementary RNA molecule which is no greater than +1°C, + 2°C, + 3°C, + 4°C, + 5°C, + 6°C, when compared to the Tₘ of a duplex between a corresponding mixed sequence oligonucleotide consisting solely of RNA and the complementary RNA molecule.

In one embodiment, the mixed sequence oligonucleotide can have a Tₘ in a duplex with a complementary RNA molecule which is no greater than - 1°C, -2°C, -3°C, -4°C, -5°C or - 6°C (i.e. does not have a Tₘ which is lower than -6°C) when compared to the Tₘ of a duplex between a corresponding mixed sequence oligonucleotide consisting solely of RNA and the complementary RNA molecule.

Example 2 provides a suitable assay for the measurement of the Tₘ of oligonucleotides duplexes. Alternatively Tₘ may be determined by using 3 µM solution of the oligonucleotide in 10 mM sodium phosphate/100 mM NaCl/ 0.1 nM EDTA, pH 7.0 is mixed with its complement DNA or RNA oligonucleotide at 3 µM concentration in 10 mM sodium phosphate/100 mM NaCl**/** 0.1 nM EDTA, pH 7.0 at 90 °C for a minute and allowed to cool down to room temperature. The melting curve of the duplex is then determined by measuring the absorbance at 260 nm with a heating rate of 1 °C/min, in the range of 25 to 95 °C. The Tₘ is measured as the maximum of the first derivative of the melting curve.

Tₘ is a measure of hybridisation, a decrease in the Tₘ is therefore equivalent to a decrease in hybridisation.

In an embodiment, the Tₘ of the duplex between the mixed sequence oligonucleotide and the complementary RNA molecule, or the double stranded oligonucleotide, is no greater than (about) 90°C, such as no greater than (about) 85°C, such as no greater than (about) 80°C, such as no greater than (about) 75°C, such as no greater than (about) 70°C.

In one embodiment, for example when the oligonucleotide according to the invention mediates RNAi, it is desirable that the Tₘ of the duplex between the mixed sequence oligonucleotide and the complementary RNA molecule, or the double stranded oligonucleotide, is about the same as the Tₘ of the equivalent unmodified RNA oligonucleotide.

In one embodiment, each strand in the double stranded oligonucleotide according to the invention is between 17 - 22 nucleotides or more preferably between 19 - 21 nucleotides in each strand.

In still another aspect the present invention relates to a pharmaceutical composition comprising a mixed sequence oligonucleotide or double stranded oligonucleotide (e.g. a modified siRNA) according to the invention and a pharmaceutically acceptable diluent, carrier or adjuvant.

In a further aspect the present invention relates to a mixed sequence oligontideotide or a double stranded oligonucleotide (e.g. a modified siRNA )according to the invention for use as a medicament.

In a still further aspect the present invention relates to the use of a mixed sequence oligonucleotide or a double stranded oligonucleotide (e.g. a modified siRNA) according to the invention for the manufacture of a medicament for the treatment of cancer, an infectious disease or an inflammatory disease.

In an even further aspect the double stranded oligonucleotide may be used in a method for treating cancer, an infectious disease, a metabolic disease, or an inflammatory disease, said method comprising administering a mixed sequence or double stranded oligonucleotide (e.g. a modified siRNA) according to the invention or a pharmaceutical composition according to the invention to a patient in need thereof.

Other aspects of the present invention will be apparent from the below description and the appended claims.

### BRIEF DESCRIPTION OF THE DRAWINGS

Figure 1 shows that LNA can increase or decrease Tₘ depending on environment.
Figure 2 shows a summery of Figure 1.
Figure 3 shows that the linkage 3' to LNA in LNA/RNA oligonucleotide modulates Tₘ. * = Tₘ with DNA complement.
Figure 4 shows that Phosphorothioate bond 3' to LNA in an otherwise RNA phosphorothioate environment reduces Tₘ.
Figure 5 shows that Phosphorothioate bond 3' to LNA in an otherwise RNA phosphorodiester environment reduces Tₘ.
Figure 6 shows that LNA enhances nuclease stability in both phosphorodiester and phosphorothioate compounds. 2-8 LNA monomers are used, in which the higher LNA content is more nuclease resistance.
Figure 7 shows that LNA/RNA/PS/PO duplexes have gene-silencing effect on target mRNA. Also, too high Tₘ reduces the gene silencing effect.
Figure 8 shows that too low Tₘ reduces the gene silencing effect.
Figure 9 shows that optimized Tₘ results in good gene silencing effect.

### DETAILED DESCRIPTION OF THE INVENTION

### Definitions

In the present context, "siRNA" or "small interfering RNA" refers to double-stranded RNA molecules from about **12** to about **35** ribonucleotides in length that are named for their ability to specifically interfere with protein expression.

The term "modified siRNA" means that at least one of the ribonucleotides in the siRNA molecule has been modified in its ribose unit, in its nitrogenous base, in its internucleoside linkage group, or combinations thereof.

The term nucleobase is used as a collective term which encompasses both nucleotides and nucleotide analogues. A nucleobase sequence is a sequence which comprises at least two nucleotides or nucleotide analogues. In one embodiment the nucleobase sequence may comprise of only nucleotides, such as DNA units, in an alternative embodiment, the nucleobase sequence may comprise of only nucleotide analogues, such as LNA units.

In the present context the term "nucleotide" means a 2-deoxyribose (DNA) monomer or a ribose (RNA) monomer which is bonded through its number one carbon to a nitrogenous base, such as adenine (A), cytosine (C), thymine (T), guanine (G) or uracil (U), and which is bonded through its number five carbon atom to an internucleoside linkage group (as defined below) or to a terminal group (as defined below).

Accordingly, when used herein the term "RNA nucleotide" or "ribonucleotide" encompasses a RNA monomer comprising a ribose unit which is bonded through its number one carbon to a nitrogenous base selected from the group consisting of A, C, G and U, and which is bonded through its number five carbon atom to a phosphate group or to a terminal group.

Analogously, the term "DNA nucleotide" or "2-deoxyribonucleotide" encompasses a DNA monomer comprising a 2-deoxyribose unit which is bonded through its number one carbon to a nitrogenous base selected from the group consisting of A, C, T and G, and which is bonded through its number five carbon atom to a phosphate group or to a terminal group.

When used herein the term "modified RNA nucleotide" or "modified ribonucleotide" means that the RNA nucleotide in question has been modified in its ribose unit, in its nitrogenous base, in its internucleoside linkage group, or combinations thereof. Accordingly, a "modified RNA nucleotide" may contain a sugar moiety which differs from ribose, such as a ribose monomer where the 2'-OH group has been modified. Alternatively, or in addition to being modified at its ribose unit, a "modified RNA nucleotide" may contain a nitrogenous base which differs from A, C, G and U (a "non-RNA nucleobase"), such as T or ^{Me}C. Finally, a "modified RNA nucleotide" may contain an internucleoside linkage group which is different from phosphate (-O-P(O)₂-O- ), such as -O-P(O,S)-O-**.**

The term "RNA nucleotide analogue" as used herein refers to any nucleotide or nucleotide analogue, other than LNA, which forms an RNA like conformation (e.g. A-form) when in a duptex with a complementary RNA nucleotide. Suitably the RNA nucleotide analogue may be a nucleotide or nucleotide analogue which has a 2' substituent group other than hydrogen.

The term "DNA nucleobase" covers the following nitrogenous bases: A, C, T and G.

The term "RNA nucleobase" covers the following nitrogenous bases: A, C, U and G.

As used herein, the "non-RNA nucleobase" encompasses nitrogenous bases which differ from A, C, G and U, such as purines (different from A and G) and pyrimidines (different from C and U).

In the present context, the term "nucleobase" includes DNA nucleobases, RNA-nucleobases and non-RNA nucleobases.

When used herein the term "sugar moiety which differs from ribose" refers to a pentose with a chemical structure that is different from ribose. Specific examples of sugar moieties which are different from ribose include ribose monomers where the 2'-OH group has been modified.

When used in the present context, the terms "locked nucleic acid monomer", "locked nucleic acid residue", "LNA monomer" or "LNA residue" refer to a bicyclic nucleotide analogue. LNA monomers are described in *inter alia* WO 99/14226, WO 00/56746, WO 00/56748, WO 01/25248, WO 02/28875, WO 03/006475 and WO 03/095467. The LNA monomer may also be defined with respect to its chemical formula. Preferred LNA monomers are described in WO 2007/031091 Thus, a "LNA monomer" as used herein has the chemical structure shown in Scheme 1 below:

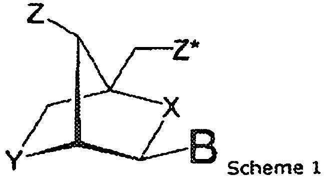

X and Y are independently selected among the groups -O-, -S-, -N(H)-, N(R)-, -CH₂- or - CH- (if part of a double bond), -CH₂-O-, -CH₂-S-, -CH₂-N(H)-, -CH₂-N(R)-, -CH₂-CH₂- or - CH₂-CH- (if part of a double bond), -CH=CH-, where R is selected form hydrogen and C₁₋₄-alkyl ; Z and Z* are independently selected among an internucleotide linkage, a terminal group or a protecting group; B constitutes a natural or non-natural nucleobase; and the asymmetric groups may be found in either orientation.

In one embodiment, X is selected from the group consisting of O, S and NR", where R^{H} is H or alkyl, such as C₁₋₄-alkyl; Y is (-CH₂) where r is an integer of 1-4; Z and Z* are independently absent or selected from the group consisting of an internucleoside linkage group, a terminal group and a protection group; and B is a nucleobase.

The term "internucleoside linkage group" is intended to mean a group capable of covalently coupling together two nucleosides, two LNA monomers, a nucleoside and a LNA monomer, etc. Specific and preferred examples include phosphate groups and phosphorothioate groups.

The term "nucleic acid" is defined as a molecule formed by covalent linkage of two or more nucleotides. The terms "nucleic acid" and "polynucleotide" are used interchangeable herein. When used herein, a "nucleic acid" or a "polynucleotide" typically contains more than 35 nucleotides.

The term "oligonucleotide" refers, in the context of the present invention, to an oligomer (also called oligo) of RNA, DNA and/or LNA monomers as well as variants and analogues thereof. When used herein, an "oligonucleotide" typically contains 2-35 nucleotides, in particular 12-35 nucleotides.

The terms "unit", "residue" and "monomer" are used interchangeably herein.

The term "at least one" encompasses an integer larger than or equal to 1, such as 1, 2, 3, 4, 5, 6, 7, 8, 9, 10, 11, 12, 13, 14, 15, 16, 17 and so forth.

The terms "a" and "an" as used about a nucleotide, an active agent, a LNA monomer, etc. is intended to mean one or more. In particular, the expression "a component (such as a nucleotide, an active agent, a LNA monomer or the like) selected from the group consisting of ..." is intended to mean that one or more of the cited components may be selected. Thus, expressions like "a component selected from the group consisting of A, B and C" is intended to include all combinations of A, B and C, i.e. A, B, C, A+B, A+C, B+C and A+B+C.

The term "thio-LNA" comprises a locked nucleobase in which at least one of X or Y in Scheme 1 is selected from S or -CH₂-S-. Thio-LNA can be in both beta-D and alpha-L-configuration. In one embodiment, X in Scheme 1 is S. Generally the beta-D.form of thio-LNA is preferred. The beta-D form of thio-LNA is shown in Scheme 3 as compound **2C**.

The term "amino-LNA" comprises a locked nucleobase in which at least one of X or Y in Scheme 1 -N(H)-, N(R)-, CH₂-N(H)-, -CH₂-N(R)- where R is selected form hydrogen and C₁-₄-alkyl. In one embodiment, "amino-LNA" refers to a locked nucleotide in which X in Scheme 1 is NH or NR", where R^{H} is hydrogen or C₁₋₄-alkyl. Amino-LNA can be in both the beta-D form and alpha-L form. Generally, the beta-D form of amino-LNA is preferred. The beta-D form of amino-LNA is shown in Scheme 2 as compound **2D**.

The term "oxy-LNA" comprises a locked nucleotide in which at least one of X or Y in Scheme 21represents -0- or -CH₂-O-. Oxy-LNA can be in both beta-D and alpha-L-configuration. In one embodiment, X in Scheme 1 is O. Oxy-LNA can be in both the beta-D form and alpha-L form. The beta-D form of oxy-LNA is preferred. The beta-D form and the alpha-L form are shown in Schemes 3 and 4 as compounds **2A** and **2B**, respectively.

The term "ena-LNA" comprises a locked nucleotide in which Y in Scheme 1 is -CH₂-O-(where the (wherein the oxygen atom of -CH₂-O- is attached to the 2-position relative to the nucleobase B).

As used herein, the term "mRNA" means the presently known mRNA transcript(s) of a targeted gene, and any further transcripts, which may be identified.

As used herein, the term "target nucleic acid" encompass any RNA that would be subject to modulation, targeted cleavage, steric blockage (decrease the abundance of the target RNA and/or inhibit translation) guided by the antisense strand. The target RNA could, for example, be genomic RNA, genomic viral RNA, mRNA or a pre-mRNA, or a miRNA or pre-miRNA.

As used herein, the term "target-specific nucleic acid modification" means any modification to a target nucleic acid.

As used herein, the term "gene" means the gene including exons, introns, non-coding 5' and 3' regions and regulatory elements and all currently known variants thereof and any further variants, which may be elucidated. In one embodiment the term 'gene' may also include miRNA or pre-miRNA.

As used herein, the term "modulation" means either an increase (stimulation) or a decrease (inhibition) in the expression of a gene or increase or decrease of the abundance of the gene product, such as replacing a non-existing or diminished microRNA in the form of an microRNA mimic for example). In the present invention, inhibition is the preferred form of modulation of gene expression and mRNA or miRNA is a preferred target.

As used herein, the term "targeting" an siLNA or siRNA compound to a particular target nucleic acid means providing the siRNA or siLNA oligonucleotide to the cell, animal or human in such a way that the siLNA or siRNA compounds are able to bind to and modulate the function of the target.

As used herein, "hybridisation" means hydrogen bonding, which may be Watson-Crick, Hoogsteen, reversed Hoogsteen hydrogen bonding, etc., between complementary nucleoside or nucleotide bases. The four nucleobases commonly found in DNA are G, A, T and C of which G pairs with C, and A pairs with T. In RNA T is replaced with uracil (U), which then pairs with A. The chemical groups in the nucleobases that participate in standard duplex formation constitute the Watson-Crick face. Hoogsteen showed a couple of years later that the purine nucleobases (G and A) in addition to their Watson-Crick face have a Hoogsteen face that can be recognised from the outside of a duplex, and used to bind pyrimidine oligonucleotides via hydrogen bonding, thereby forming a triple helix structure.

In the context of the present invention "complementary" refers to the capacity for precise pairing between two nucleic acid sequences (such as oligonucleotide) with one another. For example, if a nucleotide at a certain position of an oligonucleotide is capable of hydrogen bonding with a nucleotide at the corresponding position of a DNA or RNA molecule, then the oligonucleotide and the DNA or RNA are considered to be complementary to each other at that position. The DNA or RNA strand are considered complementary to each other when a sufficient number of nucleotides in the oligonucleotide can form hydrogen bonds with corresponding nucleotides in the target DNA or RNA to enable the formation of a stable complex. To be stable *in vitro* or *in vivo* the sequence of a siLNA or siRNA compound need not be 100% complementary to its target nucleic acid. The terms "complementary" and "specifically hybridisable" thus imply that the siRNA or siRNA compound binds sufficiently strong and specific to the target molecule to provide the desired interference with the normal function of the target whilst leaving the function of non-target mRNAs unaffected

In the present context the term "conjugate" is intended to indicate a heterogenous molecule formed by the covalent attachment of a compound as described herein to one or more non-nucleotide or non-polynucleotide moieties. Examples of non-nucleotide or non-polynucleotide moieties include macromolecular agents such as proteins fatty acid, chains, sugar residues, glycoproteins, polymers, or combinations thereof. Typically proteins may be antibodies for a target protein. Typical polymers may be polyethelene glycol.

In the present context, the term "C₁₋₆-alkyl" is intended to mean a linear or branched saturated hydrocarbon chain wherein the longest chains has from one to six carbon atoms, such as methyl, ethyl, n-propyl, isopropyl, n-butyl, isobutyl, sec-butyl, tert-butyl, pentyl, isopentyl, neopentyl and hexyl. A branched hydrocarbon chain is intended to mean a C₁₋₆-alkyl substituted at any carbon with a hydrocarbon chain.

In the present context, the term "C₁₋₄-alkyl" is intended to mean a linear or branched saturated hydrocarbon chain wherein the longest chains has from one to four carbon atoms, such as methyl, ethyl, n-propyl, isopropyl, n-butyl, isobutyl, sec-butyl and tert-butyl. A branched hydrocarbon chain is intended to mean a C₁₋₄-alkyl substituted at any carbon with a hydrocarbon chain.

When used herein the term "C₁₋₆-alkoxy" is intended to mean C₁₋₆-alkyl-oxy, such as methoxy, ethoxy, n-propoxy, isopropoxy, n-butoxy, isobutoxy, sec-butoxy, tert-butoxy, pentoxy, isopentoxy, neopentoxy and hexoxy.

In the present context, the term "C₂₋₆-alkenyl" is intended to mean a linear or branched hydrocarbon group having from two to six carbon atoms and containing one or more double bonds. Illustrative examples of C₂₋₆-alkenyl groups include allyl, homo-allyl, vinyl, crotyl, butenyl, butadienyl, pentenyl, pentadienyl, hexenyl and hexadienyl. The position of the unsaturation (the double bond) may be at any position along the carbon chain.

In the present context the term "C₂₋₆-alkynyl" is intended to mean linear or branched hydrocarbon groups containing from two to six carbon atoms and containing one or more triple bonds. Illustrative examples of C₂₋₆-alkynyl groups include acetylene, propynyl, butynyl, pentynyl and hexynyl. The position of unsaturation (the triple bond) may be at any position along the carbon chain. More than one bond may be unsaturated such that the "C₂₋₆-alkynyl" is a di-yne or enedi-yne as is known to the person skilled in the art.

### Compounds of the Invention

The embodiments referred to below with respect to the oligonucleotide according to the invention apply to a double stranded oligonucleotide, and to independently each individual strand which makes up the double stranded oligonucleotide.

### LNA Units

The LNA unit(s) may be selected from the group consisting of thio-LNA, amino-LNA, oxy-LNA and ena-LNA. These LNAs have the general chemical structure shown in Scheme 1b below:

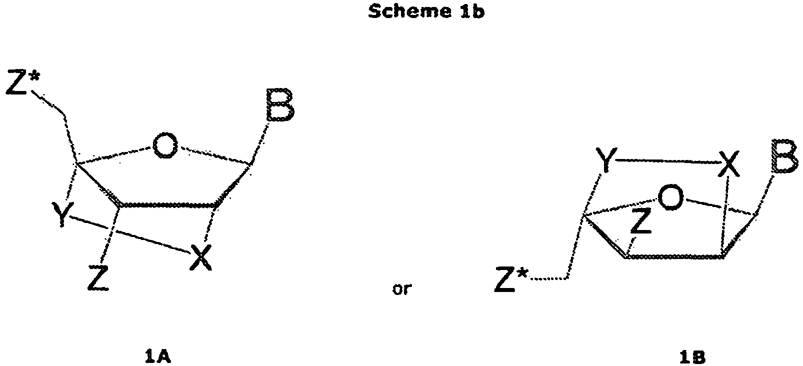

Wherein X is selected from the group consisting of O, S and NR", where R^{H} is H or alkyl, such as C₁₋₄-alkyl; Y is (-CH₂)ᵣ, where r is an integer of 1-4; Z and Z* are independently absent or selected from the group consisting of an internucleoside linkage group, a terminal group and a protection group; and B is a nucleobase.

In a preferred embodiment of the invention, r is 1, i.e. a preferred LNA monomer has the chemical structure shown in Scheme 2 below: 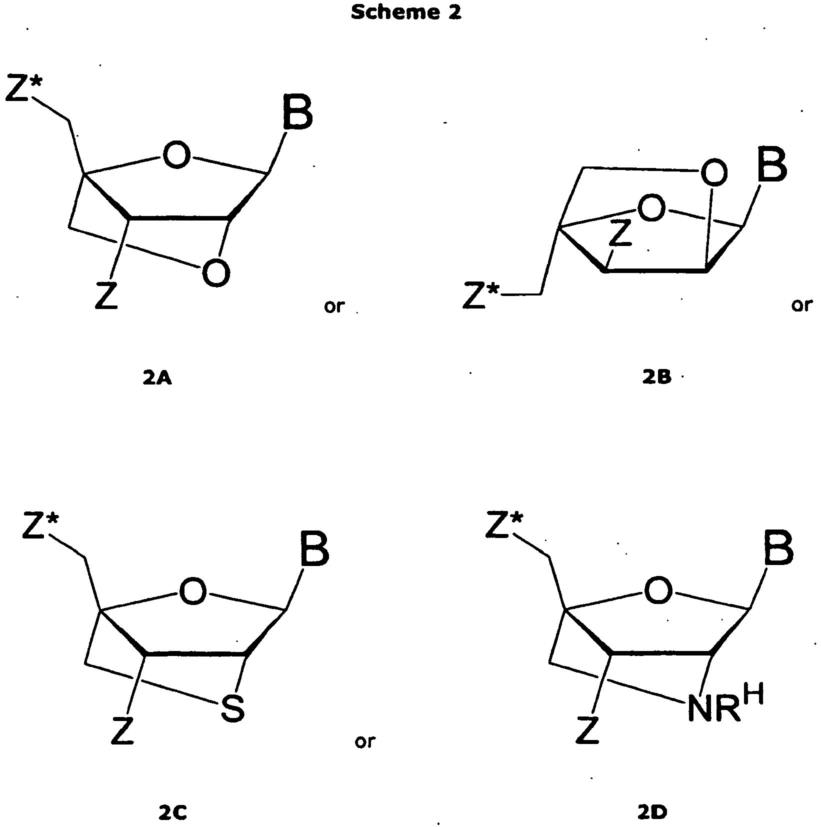 wherein Z, Z*, R^{H} and B are defined above.

In an even more preferred embodiment of the invention, X is O and r is 1, i.e. an even more preferred LNA monomer has the chemical structure shown in Scheme 3 below: 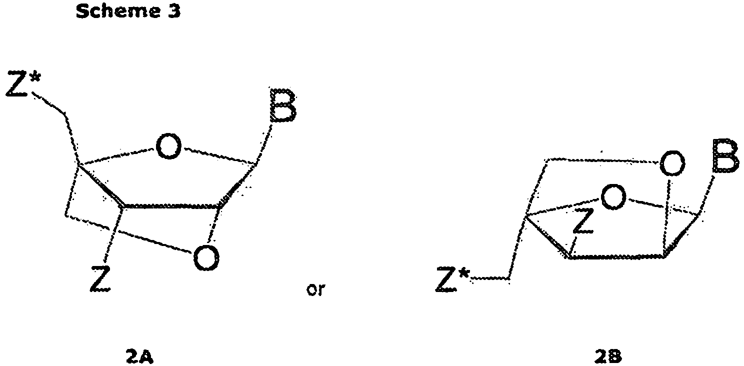 wherein Z, Z* and B are defined above.

The structures shown in **2A** and **2B** above may also be referred to as the "beta-D form" and the "alpha-L form", respectively. In a highly preferred embodiment of the invention, the LNA monomer is the beta-D form, i.e. the LNA monomer has the chemical structure indicated in **2A** above, such as beta-D-oxy or beta-D-amino

As indicated above, Z and Z*, which serve for an internucleoside linkage, are independently absent or selected from the group consisting of an internucleoside linkage group, a terminal group and a protection group depending on the actual position of the LNA monomer within the compound. It will be understood that in embodiments where the LNA monomer **is** located at the **3'** end, Z is a terminal group and Z* is an internucleoside linkage. In embodiments where the LNA monomer is located at the 5' end, Z is absent and Z* is a terminal group. In embodiments where the LNA monomer is located within the nucleotide sequence, Z is absent and Z* is an internucleoside linkage group.

### Internucleoside Linkages

The mixed sequence oligonucleotide according to the invention is characterised in that it comprises at least one dinucleotide of sequence 5' LNA-PS-XNA 3', wherein; XNA is either an RNA nucleotide or an RNA nucleotide analogue;LNA is a locked nucleic acid; and PS is a phosphorothioate internucloside linkage O P(O,S)-O-, and the mixed sequence oligonucleotide comprises both phosphorothioate and phosphodiestet linkages.

The remaining internucleoside linkages may be selected from the group consisting of: -OP(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-, -O-PO(OCH₂CH₂S-R)-O-, -O-PO(BH₃)-O-, -O-PO(NHR^{H})-O-, -O-P(O)₂-NR^{H}-, **-**NR^{H}**-**P(O)₂-**O-,** -NR^{H}-CO-O-, -NR^{H}-CO-NR^{H}-, -O-CO-O-, -O-CO-NR^{H}-, -NR^{H}-CO-CH₂-, -O-CH₂-CO-NR^{H}-, - O-CH₂-CH₂-NR^{H}-, -CO-NR^{H}-CH₂-, -CH₂-NR^{H}-CO-, -O-CH₂-CH₂-S-, **-S-CH₂-CH₂-O-,** -S-CHa-CH₂-S-, -CH₂-SO₂-CH₂-, -CH₂-CO-NR^{H}-, -O-CH₂-CH₂-NR^{H}-CO -, -CH₂-NCH₃-O-CH₂-, where R^{H} is hydrogen or C₁₋₄-alkyl.

In one embodiment the remaining internucleoside linkages are selected form the group consisting of phosphorothioate, phosphodiester and phosphate.

In one embodiment, the remaining internucleoside linkages are phosphodiester linkages.

In one embodiment, the remaining Internucleoside linkages are phosphorothioate linkages.

In one embodiment, the remaining **internucleoside** linkages are phosphate linkages.

In one embodiment, at least 1, 2, 3, 4, 5, 6, 7, 8, 9, 10, 11, 12, 13, 14, 15, 16, 17, 18, 19, 20, of the remaining internucleoside linkages are phosphodiester linkages.

In one embodiment, at least 1, 2, 3, 4, 5, 6, 7, 8, 9, 10, 11, 12, 13, 14, 15, 16, 17, 18, 19, 20, of the remaining internucleoside linkages are phosphorothioate linkages.

In one embodiment, at least 1, 2, 3, 4, 5, 6, 7, 8, 9, 10, 11, 12, 13, 14, 15, 16, 17, 18, 19, 20, of the remaining internucleoside linkages are phosphate linkages.

The mixed sequence oligonucleotide according to the invention comprises both phosphate groups and phosphorothioate groups.

In one embodiment the remaining internucleoside groups are phosphorothioate and/or phosphodiester linkages.

In one embodiment, phosphorothioate linkage between the 5'LNA and 3'XNA of the diunucleotide of the mixed sequence oligonucleotide is the only phosphorothioate linkage in the oligonucleotide

### Terminal Groups

Specific examples of terminal groups include terminal groups selected from the group consisting of hydrogen, azido, halogen, cyano, nitro, hydroxy, Prot-O-, Act-O-, mercapto, Prot-S-, Act-S-, C₁₋₆-alkylthio, amino, Prot-N(R^{H})-, Act-N(R^{H})-, mono- or di(C₁₋₆-alkyl)amino, optionally substituted C₁₋₆- alkoxy, optionally substituted C₁₋₆-alkyl, optionally substituted C₂₋₆-alkenyl, optionally substituted C₂₋₆-alkenyloxy, optionally substituted C₂₋₆-alkynyl, optionally substituted C₂₋₆-alkynyloxy, monophosphate including protected monophosphate, monothiophosphate including protected monothiophosphate, diphosphate including protected diphosphate, dithiophosphate including protected dithiophosphate, triphosphate including protected triphosphate, trithiophosphate including protected trithiophosphate, where Prot is a protection group for -OH, -SH and -NH(R^{H}), and Act is an activation group for -OH, -SH, and -NH(R^{H}), and R^{H} is hydrogen or C₁₋₆-alkyl.

Examples of phosphate protection groups include S-acetylthioethyl (SATE) and S-pivaloylthioethyl (t-butyl-SATE).

Still further examples of terminal groups include DNA intercalators, photochemically active groups, thermochemically active groups, chelating groups, reporter groups, ligands, carboxy, sulphono, hydroxymethyl, Prot-O-CH₂-, Act-O-CH₂-, aminomethyl, Prot-N(R^{H})-CH₂-, Act-N(R^{H})-CH₂-, carboxymethyl,sulphonomethyl, where Prot is a protection group for -OH, -SH and -NH(R^{H}), and Act is an activation group for -OH, -SH, and -NH(R^{H}), and R^{H} is hydrogen or C₁₋₆-alkyl.

### Protection Groups

Examples of protection groups for -OH and -SH groups include substituted trityl, such as 4,4'-dimethoxytrityloxy (DMT), 4-monomethoxytrityloxy (MMT); trityloxy, optionally substituted 9-(9-phenyl)xanthenyloxy (pixyl), optionally substituted methoxytetrahydropyranyloxy (mthp); silyloxy, such as trimethylsilyloxy (TMS), triisopropylsilyloxy (TIPS), tert-butyldimethylsilyloxy (TBDMS), triethylsilyloxy, phenyldimethylsilyloxy; *tert-*butylethers; acetals (including two hydroxy groups); acyloxy, such as acetyl or halogen-substituted acetyls, *e*.*g*. chloroacetyloxy or fluoroacetyloxy, isobutyryloxy, pivaloyloxy, benzoyloxy and substituted benzoyls, methoxymethyloxy (MOM), benzyl ethers or substituted benzyl ethers such as 2,6-dichlorobenzyloxy (2,6-Cl₂Bzl). Moreover, when Z or Z* is hydroxyl they may be protected by attachment to a solid support, optionally through a linker.

Examples of amine protection groups include fluorenylmethoxycarbonylamino (Fmoc), *tert-*butyloxycarbonylamino (BOC), trifluoroacetylamino, allyloxycarbonylamino (alloc, AOC), Z-benzyloxycarbonylamino (Cbz), substituted benzyloxycarbonylamino, such as 2-chloro benzyloxycarbonylamino (2-CIZ), monomethoxytritylamino (MMT), dimethoxytritylamino (DMT), phthaloylamino, and 9-(9-phenyl)xanthenylamino (pixyl).

### Activation Groups

The activation group preferably mediates couplings to other residues and/or nucleotide monomers and after the coupling has been completed the activation group is typically converted to an internucleoside linkage. Examples of such activation groups include optionally substituted O-phosphoramidite, optionally substituted O-phosphortriester, optionally substituted O-phosphordiester, optionally substituted H-phosphonate, and optionally substituted O-phosphonate. In the present context, the term "phosphoramidite" means a group of the formula -P(OR^{x})-N(R^{y})₂, wherein R^{x} designates an optionally substituted-alkyl group, *e.g*. methyl, 2-cyanoethyl, or benzyl, and each of R^{y} designates optionally substituted alkyl groups, e.g. ethyl or isopropyl, or the group -N(R^{y})₂ forms a morpholino group (-N(CH₂CH₂)₂O), R^{x} preferably designates 2-cyanoethyl and the two R^{y} are preferably identical and designates isopropyl. Accordingly, a particularly preferred phosphoramidite is N,N-diisopropyl-O-(2-cyanoethyl)phosphoramidite.

As indicated above, B is a nucleobase which may be of natural or non-natural origin Specific examples of nucleobases include adenine (A), cytosine (C), 5-methylcytosine (^{Me}C), isocytosine, pseudoisocytosine, guanine (G), thymine (T), uracil (U), 5-bromouracil, 5-propynyluracil, 5-propyny-6, 5-methylthiazoleuracil, 6-aminopurine, 2-aminopurine, inosine, 2,6-diaminopurine, 7-propyne-7-deazaadenine, 7-propyrre-7-deazaguanine and 2-chloro-6-aminopurine.

### RNA Analogues

In a preferred embodiment XNA is a RNA nucleotide.

However, it is also envisaged that XNA may be an RNA analogue, other than LNA. Suitably RNA analogues which comprise a 2' substitution may also be used. In one embodiment the 2' substitution is with a halogen, such as fluorine (2'Flouro). Preferable 2' substitutions include substitutions with oxygen containing side groups, *i.e*. a 2' O substituent, such as 2'Oalkyl (such as 2'Omethyl) or 2'Ometoxyethyl. The alkyl group may for example be between C₁-C₄ or C₁-C₆, such as C₁, C₂, C₃, C₄, C₅ or C₆. Therefore, in one embodiment, the term RNA analogue are nucleotides which consists of a 2' subsistent selected from the group consisting of 2'halo, such as 2'fluoro, and a 2' O substituent, such as 2'Omethyl or 2'Ometoxyethyl. In the context of the present invention LNA is not an RNA analogue. It will be recognised, where suitable, that such modifications can be in alternative stereochemical forms, for example, the 2'fluoro substituent may be in either arabino- or ribo-.configuration.

### Combined and further modifications

As will be understood by the skilled person, any of the above-mentioned modifications may be combined and/or the oligonculeotide of the invention may contain other modifications which serve the purpose of modulating the biostability, increasing the nuclease resistance, improving the cellular uptake and/or improving the tissue distribution.

### 5' LNA-PS-XNA 3' dinucleotide

The oligonucleotide according to the invention comprises at least one 5' LNA-PS-XNA 3' dinucleotide.

However, it is envisaged that the oligonucleotide according to the invention may comprise more than one 5' LNA-PS-XNA 3' dinucleotide, such as (at least) two 5' LNA-PS-XNA 3' dinucleotides, such as (at least) three 5' LNA-PS-XNA 3' dinucleotides, such as (at least) 4 5' LNA-PS-XNA 3' dinucleotides, such as (at least) five 5' LNA-PS-XNA 3' dinucleotides, such as (at least) six 5' LNA-PS-XNA 3' dinucleotides, (such as) at least seven 5' LNA-PS-XNA 3' dinucleotides, such as (at least) 8 5' LNA-PS-XNA 3' dinucleotides, such as (at least) 9 5' LNA-PS-XNA 3' dinucleotides, such as (at least) 10 5' LNA-PS-XNA 3' dinucleotides.

In one embodiment, the oligonucleotide of the invention comprise a sequence of (5' LNA-PS-XNA 3')_{q}, where q is an integer between 1 and 12, such as 2, 3, 4, 5, 6, 7, 8, 9, 10 and 11.

### Other Nucleobases

In conjunction with the at least one 5' LNA-PS-XNA 3' dinucleotide, the oligonucleotide according to the invention comprises a sequence of nucleobases which has a mixed sequence.

The other nucleobases (other than the dinucleotide) may be selected independently from the group consisting of DNA, DNA analogues, RNA, RNA analogues, LNA.

In one preferred embodiment the remaining nucleobases are all RNA nucleotides.

The oligonucleotide according to the invention therefore may comprise at least 1 (further) RNA nucleotide, such as (at least) 2 RNA nucleotides. such as (at least) 3 RNA nucleotides, such as (at least) 4 RNA nucleotides, such as (at least) 5 RNA nucleotides, such as (at least) 6 RNA nucleotides, such as (at least) 7 RNA nucleotides, such as (at least) 8 RNA nucleotides, such as (at least) 9 RNA nucleotides, such as (at least) 10 RNA nucleotides, such as (at least) 11 RNA nucleotides, such as (at least) 12 RNA nucleotides, such as (at least) 13 RNA nucleotides, such as (at least) 14 RNA nucleotides, such as (at least) 15 RNA nucleotides, such as (at least) 16 RNA nucleotides, such as (at least) 17 RNA nucleotides, such as (at least) 18 RNA nucleotides, such as (at least) 19 RNA nucleotides, such as (at least) 20 RNA nucleotides, such as (at least) 21 RNA nucleotides, such as (at least) 22 RNA nucleotides, such as 23 RNA nucleotides,

In one embodiment the remaining nucleobases are all DNA nucleotides.

The oligonucleotide according to the invention therefore may comprise at least 1 (further) DNA nucleotide, such as (at least) 2 DNA nucleotides. such as (at least) 3 DNA nucleotides, such as (at least) 4 DNA nucleotides, such as (at least) 5 DNA nucleotides, such as (at least) 6 DNA nucleotides, such as (at least) 7 DNA nucleotides, such as (at least) 8 DNA nucleotides, such as (at least) 9 DNA nucleotides, such as (at least) 10 DNA nucleotides, such as (at least) 11 DNA nucleotides, such as (at least) 12 DNA nucleotides, such as (at least) 13 DNA nucleotides, such as (at least) 14 DNA nucleotides, such as (at least) 15 DNA nucleotides, such as (at least) 16 DNA nucleotides, such as (at least) 17 DNA nucleotides, such as (at least) 18 DNA nucleotides, such as (at least) 19 DNA nucleotides, such as (at least) 20 DNA nucleotides, such as (at least) 21 DNA nucleotides, such as (at least) 22 DNA nucleotides, such as 23 DNA nucleotides,

It is known that LNA monomers incorporated into oligos will induce a RNA-like structure of the oligo and the hybrid that it may form. It has also been shown that LNA residues modify the structure of DNA residues, in particular when the LNA residues are incorporated in the proximity of 3'-end. LNA monomer incorporation towards the 5'-end seems to have a smaller effect. This means that it is possible to modify RNA strands which contain DNA monomers, and if one or more LNA residues flank the DNA monomers they too will attain a RNA-like structure. Therefore, DNA and LNA monomers can replace RNA monomers and still the oligo will attain an overall RNA-like structure. As DNA monomers are considerably cheaper than RNA monomers, easier to synthesise and more stable towards nucleolytic degradation, such modifications will therefore improve the overall use and applicability of siRNAs.

Therefore In one embodiment, the further nucleobases consist of LNA and DNA residues, such as alternate LNA and DNA residues. It is envisaged that within the spirit of such an embodiment, an equivalent exists where other nucleotide analogues (DNA analogues), or even one or two RNA units may be used in place of the DNA units. It is also envisaged that RNA analogues, several of which are equivalent to 2' modified DNA units, may also be used in place of one or more of the DNA units.

It is envisaged that the use of at least one or more further nucleotide analogues may be preferable, particularly LNA. The further LNA nucleobases may, in one embodiment be in the form of further 5' LNA-PS-XNA 3' dinucleotides or they may be outside of the context of a 5' LNA-PS-XNA 3' dinucleotide.

The oligonucleotide according to the invention therefore may comprise at least 1 (further) LNA nucleobase, such as (at least) 2 LNA nucleobases. such as (at least) 3 LNA nucleobases, such as (at least) 4 LNA nucleobases, such as (at least) 5 LNA nucleobases, such as (at least) 6 LNA nucleobases, such as (at least) 7 LNA nucleobases, such as (at least) 8 LNA nucleobases, such as (at least) 9 LNA nucleobases, such as (at least) 10 LNA nucleobases, such as (at least) 11 LNA nucleobases, such as (at least) 12 LNA nucleobases, such as (at least) 13 LNA nucleobases, such as (at least) 14 LNA. nucleobases, such as (at least) 15 LNA nucleobases, such as (at least) 16 LNA nucleobases, such as (at least) 17 LNA nucleobases, such as (at least) 18 LNA nucleobases, such as (at least) 19 LNA nucleobases, such as (at least) 20 LNA nucleobases, such as (at least) 21 LNA nucleobases, such as (at least) 22 LNA nucleobases, such as 23 LNA nucleobases,

In one embodiment at least 10%, such as at least 20%, such as at least 30%, such as at least 40%, such as at least 50% of the nucleobases in the oligonucleotide according to the invention are LNA units.

In one embodiment at least 10%, such as at least 20%, such as at least 30%, such as at least 40%, such as at least 50% of the nucleobases in the oligonucleotide according to the invention are DNA units.

In one embodiment at least 10%, such as at least 20%, such as at least 30%, such as at least 40%, such as at least 50% of the nucleobases in the oligonucleotide according to the invention are RNA units.

In one embodiment up to 80%, such as up to 75%, such as up to 70%, such as up to 60%, such as up to 50%, such as up to 40%, such as up to 30%, such as up to 20% of the nucleobases the oligonucleotide according to the invention are LNA units.

In one embodiment between 1 - 20, such as between 1 - 12 of the nucleobases in the oligonucleotide of the invention are LNA units.

In one embodiment between 1 - 6 of the nucleobases in the oligonucleotide of the invention are LNA units.

In one embodiment the central nucleobase, or, at least one, or both of the central nucleobases are LNA units.

Therefore, in a highly interesting embodiment of the invention, the oligonculeotide of the invention, such as double stranded oligonucleotide of the invention further comprises at least one modified RNA nucleotide. This further modification or modifications may be a modification selected from the group consisting of a non-RNA nucleobase, a sugar moiety which differs from ribose, an internucleoside linkage group which differs from phosphate, and combinations thereof. As will be understood, selection of preferred non-RNA nucleobases, preferred sugar moieties which differ from ribose, and preferred internucleotide linkage groups which differ from phosphate will be the same as those described in the sections entitled "Modification of the nucleobase", "Modification of the sugar moiety" and "Modification of the internucleoside linkage group". For example, in one embodiment of the invention, the oligonucleotide of the invention, such as a first (sense) strand comprises at least one LNA monomer, such as 1-10 LNA monomers, e.g. 1-5 or 1-3 LNA monomers. In another embodiment (or the same embodiment) of the invention, the second (antisense) strand comprises at least one LNA monomer, such as 1-10 LNA monomers, e.g. 1-5 or 1-3 LNA monomers. In a further embodiment of the invention, the first strand comprises at least one LNA monomer and the second strand comprises at least one LNA monomer. For example, the first strand typically comprises 1-10 LNA monomers, such as 1-5 or 1-3 LNA monomers, and the second strand typically comprises 1-10 LNA monomers, such as 1-5 or 1-3 ULNA monomers.

### Length of the Oligonucleotide

In one embodiment, the oligonucleotide has a length of 15 - 25 nucleobases.

In one embodiment, the oligonucleotide has a length of 15 - 20 nucleobases.

In one embodiment, the oligonucleotide has a length of 15 - 18 nucleobases.

In one embodiment, the oligonucleotide has a length of 15, 16, 17, 18, 19, 20, 21, 22, 23, 24 or 25 nucleobases.

In one embodiment, the oligonucleotide is between 12 and 17, such as between 13 and 16, such as 14 or 15 nucleobases in length.

### siRNAs

Due to the ability to reduce the Tₘ of LNA containing oligonucleotides, whilst retaining the stabilisation and nuclease protection conferred by the LNA units, the 5' LNA-PS-XNA 3' duplex is particularly suited to single stranded or double stranded oligonucleotides for the mediation of RNAi, or similar silencing mechanisms where the efficacy is dependant upon the separation of the two oligonucleotide strands, or the oligonucleotide form the target molecule.

While LNA monomers can be used freely in the design of modified siLNAs at both 3'-overhangs and at the 5'-end of the sense strand with full activation of the siLNA effect and down-regulation of protein production, the present inventors have surprisingly found that the mRNA-cleaving capability of an activated RISC complex can be suppressed by modifying the sense strand of a siRNA in certain specific positions.

If the LNA monomers are incorporated in the siRNA in such a way that they are strengthening, the base pairs in the duplex at the 5'-end of the sense strand, the helicase can thereby be directed to unwinding from the other 5'-end (antisense strand 5'-end). In this way the incorporation of the antisense/guiding strand into RISC can be controlled. The helicase starts unwinding the siRNA duplex at the weakest binding end. The release 3'- end is probably targeted for degradation while the remaining strand is incorporated in the RISC. Efficient siRNAs show accumulation of the antisense/guiding strand and weaker base pairing in the 5'-end of the antisense/guiding strand. Unwanted side effects may possibly be avoided by having only the correct strand (the antisense/guiding strand) in RISC and not the unwanted sense strand (not complementary to the desired target RNA).

We have surprisingly discovered that even if only one strand of a double stranded oligonucleotide comprises a 5' LNA-PS-XNA 3', the Tₘ of the double stranded oligonucleotide can be significantly reduced, and this appears to be irrespective of whether the remaining linkages are phosphorothioate or not. For example the other strand may comprise phosphodiester or phosphate bonds, but the inclusion of a single 5' LNA-PS-XNA 3' can cause a remarkable reduction in the Tₘ.

The following embodiments are particularly of relevance to the double stranded oligonucleotide (such as the siLNA) according to the invention, although may also be of relevance to the single stranded oligonucleotide according to the invention:

When we refer to the first strand, it is considered that this is equivalent to the sense strand of an siRNA which is not targeting the mRNA (sometimes also called passenger strand), and the second strand is the 'antisense strand' and the strand which is incorporated into RISC (in the case of siRNA, sometimes also called passenger strand) and is complementary to the target mRNA. However a double stranded oligonucleotide according to the invention may serve as a miRNA mimic for replacement of the missing miRNA. In this case the antisense strand would be the miRNA copy which goes into RISC to identify the target mRNA.

In one embodiment the first strand comprises at least one 5' LNA-PS-XNA 3' dinucleotide, and the second strand does not comprise a 5' LNA-PS-XNA 3' dinucleotide.

In one embodiment the second strand comprises at least one 5' LNA-PS-XNA 3' dinucleotide, and the first strand does not comprise a 5' LNA-PS-XNA 3' dinucleotide.

In one embodiment both the first and strands both comprises at least one 5'LNA-PS-XNA 3' dinucleotide.

In one embodiment at least one (such as one) LNA monomer is located at the 5'-end of the first (e.g. sense) strand. Preferably, at least two (such as two) LNA monomers are located at the 5'-end of the first strand.

In a preferred embodiment of the invention, the first strand comprises at least one (such as one) LNA monomer located at the 3'-end of the first strand. More preferably, at least two (such as two) LNA monomers are located at the 3'-end of the of the first strand.

In a particular preferred embodiment of the invention, the first strand comprises at least one (such as one) LNA monomer located at the 5'-end of the first strand and at least one (such as one) LNA monomer located at the 3'-end of the first strand. Even more preferably, the first strand comprises at least two (such as two) LNA monomers located at the 5'-end of the first strand and at least two (such as two) LNA monomers located at the 3'-of the first strand.

It is preferred that at least one (such as one) LNA monomer is located at the 3'-end of the second (e.g. antisense) strand. More preferably, at least two (such as two) LNA monomers are located at the 3'-end of the second strand. Even more preferably, at least three (such as three) LNA monomers are located at the 3'-end of the second strand. In a particular preferred embodiment of the invention, no LNA monomer is located at or near (i.e. within 1, 2, or 3 nucleotides) the 5'-end of the second strand.

In a highly preferred embodiment of the invention, the first strand comprises at least one LNA monomer at the 5'-end and at least one LNA monomer at the 3'-end, and the second strand comprises at least one LNA monomer at the 3'-end. More preferably, the first strand comprises at least one LNA monomer at the 5'-end and at least one LNA monomer at the 3'-end, and the second strand comprises at least two LNA monomers at the 3'-end. Even more preferably, the first strand comprises at least two LNA monomers at the 5'-end and at least two LNA monomers at the 3'-end, and the second strand comprises at least two LNA monomers at the 3'-end. Still more preferably, the first strand comprises at least two LNA monomers at the 5'-end and at least two LNA monomers at the 3'-end, and the second strand comprises at least three LNA monomers at the 3'-end. It will be understood that in the most preferred embodiment, none of the above-mentioned compounds contain a LNA monomer which is located at the 5'-end of the second (e.g. antisense) strand.

In a further interesting embodiment of the invention, the LNA monomer is located close to the 3'-end of the oligonucleotide, i.e. at position 2,3 or 4, preferably at position 2 or 3, in particular at position 2, calculated from the 3'-end.

Accordingly, in a further very interesting embodiment of the invention, the first strand comprises a LNA monomer located at position 2, calculated from the 3'-end. In another embodiment, the first strand comprises LNA monomers located at position 2 and 3, calculated from the 3'-end.

In a particular preferred embodiment of the invention, the first strand comprises at least one (such as one) LNA monomer located at the 5'-end and a LNA monomer located at position 2 (calculated from the 3'-end). In a further embodiment, the first strand comprises at least two (such as two) LNA monomers located at the 5'-end of the first strand a LNA monomer located at positions 2 (calculated from the 3' end).

Furthermore, it is preferred that the second strand comprises a LNA monomer at position 2, calculated from the 3'-end. More preferably, the second strand comprises LNA monomers in position 2 and 3, calculated from the 3'-end. Even more preferably, the second strand comprises LNA monomers located at position 2, 3 and 4, calculated from the 3'-end. In a particular preferred embodiment of the invention, no LNA monomer is located at or near (i.e. within 1, 2, or 3 nucleotides) the 5'-end of the second strand.

In a highly preferred embodiment of the invention, the first strand comprises at least one LNA monomer at the 5'-end and a LNA monomer at position 2 (calculated from the 3' end), and the second strand comprises a LNA monomer located at position 2 (calculated from the 3'-end). More preferably, the first strand comprises at least one LNA monomer at the 5'-end and a LNA monomer at position 2 (calculated from the 3'-end), and the second strand comprises LNA monomers at position 2 and 3 (calculated from the 3'-end). Even more preferably, the first strand comprises at least two LNA monomers at the 5'-end and LNA monomers at position 2 and 3 (calculated from the 3'-end), and the second strand comprises LNA monomers at position 2 and 3 (calculated from the 3'-end). Still more preferably, the first strand comprises at least two LNA monomers at the 5'-end and LNA monomers at position 2 and 3 (calculated from the 3'-end), and the second strand comprises LNA monomers at position 2, 3 and 4 (calculated from the 3'-end). It will be understood that in the most preferred embodiment, none of the above-mentioned compounds contain a LNA monomer which is located at the 5'-end of the second strand.

As indicated above, each strand typically comprises 12-35 monomers. It will be understood that these numbers refer to the total number of naturally occurring and modified nucleotides. Thus, the total number of naturally occurring and modified nucleotides will typically not be lower than 12 and will typically not exceed 35. In an interesting embodiment of the invention, each strand comprises 17-25 monomers, such as 20-22 or 20-21 monomers.

The double stranded oligonucleotide according to the invention may be blunt ended or may contain overhangs. Preferably at least one of the strands comprises a 3'-overhang. In one embodiment of the invention the first and second strand both comprise a 3'-overhang. In another embodiment of the invention only the first strand comprises a 3'-overhang.

Typically, the 3'-overhang is 1-7 monomers in length, preferably 1-5 monomers in length, such as 1-3 monomers in length, e.g. 1 monomer in length, 2 monomers in length or 3 monomers in length.

In a similar way, at least one of the strands may have a 5'-overhang. Typically, the 5'-overhang will be of 1-7 monomers in length, preferably 1-3, such as 1, 2 or 3, monomers in length. Thus, it will be understood that the first strand may contain a 5'-overhang, the antisense strand may contain a 5'-overhang, or both of the first and second strands may contain 5'-overhangs. Evidently, the first strand may contain both a 3'- and a 5'-overhang. Alternatively, the second strand may contain both a 3'- and a 5'-overhang.

As far as the **LNA** monomers are concerned, it will be understood that any of the LNA monomers shown in Scheme 2 and 3 are useful for the purposes of the present invention. However, it is currently preferred that the LNA monomer is in the beta-D form, corresponding to the **LNA** monomers shown as compounds **2A, 2C** and **2D.** The currently most preferred LNA monomer is the monomer shown as compound 2A in Schemes 2 and 3 above, i.e. the currently most preferred LNA monomer is the beta-D form of oxy-LNA.

In a further embodiment of the invention, the double stranded oligonucleotide according to the invention is linked to one or more ligands so as to form a conjugate. The ligand(s) serve(s) the role of increasing the cellular uptake of the conjugate relative to the nonconjugated compound. This conjugation can take place at the terminal 5'-OH and/or 3'-OH positions, but the conjugation may also take place at the sugars and/or the nucleobases. In particular, the growth factor to which the antisense oligonucleotide may be conjugated, may comprise transferrin or folate. Transferrin-polylysine-oligonucleotide complexes or folate-polylysine-oligonucleotide complexes may be prepared for uptake by cells expressing high levels of transferrin or folate receptor. Other examples of conjugates/lingands are cholesterol moieties, duplex intercalators such as acridine, poly-L-lysine, "end-capping" with one or more nuclease-resistant linkage groups such as phosphoromonothioate, and the like.

The preparation of transferrin complexes as carriers of oligonucleotide uptake into cells is described by Wagner et al, Proc. Natl. Acad. Sci. USA 87, 3410-3414 (1990). Cellular delivery of folate-macromolecule conjugates via folate receptor endocytosis, including delivery of an antisense oligonucleotide, is described by Low et al, US 5,108,921 and by Leamon et al., Proc. Natl. Acad. Sci. 88, 5572 (1991).

The compounds or conjugates of the invention may also be conjugated or further conjugated to active drug substances, for example, aspirin, ibuprofen, a sulfa drug, an antidiabetic, an antibacterial agent, a chemotherapeutic agent or an antibiotic.

The invention further provides for a method for decreasing the Tₘ of a duplex between a mixed sequence oligonucleotide and a complementary oligonucleotide or nucleic acid sequence, said method comprising replacing at least one dinucleobase sequence present in the mixed sequence oligonucleotide with at least one dinucleotide of sequence 5' LNA-PS-XNA 3', wherein; XNA is either an RNA nucleotide or an RNA nucleotide analogue; LNA is a locked nucleic acid; and PS is a phosphorothioate internucloside linkage -O-P(O,S)-O-. The sequence of the mixed sequence oligonucleotide is retained.

The mixed sequence oligonucleotide as referred to in the above method may be as according to the mixed sequence oligonucleotide of the invention, with the proviso that prior to performing the above method, the mixed sequence oligonucleotide may, in one embodiment not comprise a dinucleotide of sequence 5' LNA-PS-XNA 3', or may comprise fewer dinucleotides of sequence 5' LNA- PS-XNA 3', than after the above method. Further more the duplex referred to above may be as according to the double stranded oligonucleotide of according to the invention, with the same proviso as referred to in the previous sentence.

### Manufacture

The oligonucleotides of the invention may be produced using the polymerisation techniques of nucleic acid chemistry, which is well known to a person of ordinary skill in the art of organic chemistry. Generally, standard oligomerisation cycles of the phosphoramidite approach (S. L. Beaucage and R. P. Iyer, Tetrahedron, 1993, 49, 6123; and S. L. Beaucage and R. P. Iyer, Tetrahedron, 1992, 48, 2223) may be used, but other chemistries, such as the H-phosphonate chemistry or the phosphortriester chemistry may also be used.

For some monomers longer coupling time and/or repeated couplings with fresh reagents and/or use of more concentrated coupling reagents may be necessary. However, in our hands, the phosphoramidites employed coupled with a satisfactory >97% step-wise coupling yield. Thiolation of the phosphate may be performed by exchanging the normal oxidation, i.e. the iodine/pyridine/H₂O oxidation, with an oxidation process using Beaucage's reagent (commercially available). As will be evident to the skilled person, other sulphurisation reagents may be employed.

Purification of the individual strands may be done using disposable reversed phase purification cartridges and/or reversed phase HPLC and/or precipitation from ethanol or butanol. Gel electrophoresis, reversed phase HPLC, MALDI-MS, and ESI-MS may be used to verify the purity of the synthesised LNA-containing oligonucleotides. Furthermore, solid support materials having immobilised thereto a nucleobase-protected and 5'-OH protected LNA are especially interesting for synthesis of the LNA-containing oligonucleotides where a LNA monomer is included at the 3' end. For this purpose, the solid support material is preferable CPG or polystyrene onto which a 3'-functionalised, optionally nucleobase protected and optionally 5'-OH protected LNA monomer is linked. The LNA monomer may be attached to the solid support using the conditions stated by the supplier for that particular solid support material.

### Therapy and Pharmaceutical Compositions

As explained initially, the oligonucleotides according to the invention will constitute suitable drugs with improved properties. Clearly, the optimisation of the design of a potent and safe drug requires the fine- tuning of diverse parameters such as affinity/specificity, stability in biological fluids, cellular uptake, mode of action, pharmacokinetic properties and toxicity.

Accordingly, in a further aspect the present invention relates to a pharmaceutical composition comprising a mixed sequence oligonucleotide or double stranded oligonucleotide (such as a modified siRNA) according to the invention and a pharmaceutically acceptable diluent, carrier or adjuvant.

In a still further aspect the present invention relates to a mixed sequence oligonucleotide or double stranded oligonucleotide according to the invention for use as a medicament.

As will be understood dosing is dependent on severity and responsiveness of the disease state to be treated, and the course of treatment lasting from several days to several months, or until a cure is effected or a diminution of the disease state is achieved. Optimal dosing schedules can be calculated from measurements of drug accumulation in the body of the patient. Optimum dosages may vary depending on the relative potency of individual molecule. Generally it can be estimated based on EC50s found to be effective in *in vitro* and *in vivo* animal models. In general, dosage is from 0.01 µg to 1 g per kg of body weight, and may be given once or more daily, weekly, monthly or yearly, or even once every 2 to 10 years or by continuous infusion for hours up to several months. The repetition rates for dosing can be estimated based on measured residence times and concentrations of the drug in bodily fluids or tissues. Following successful treatment, it may be desirable to have the patient undergo maintenance therapy to prevent the recurrence of the disease state.

### Pharmaceutical Composition

It should be understood that the invention also relates to a pharmaceutical composition, which comprises at least one mixed sequence oligonucleotide or double stranded oligonucleotide according to the invention as an active ingredient. It should be understood that the pharmaceutical composition according to the invention optionally comprises a pharmaceutical carrier, and that the pharmaceutical composition optionally comprises further compounds, such as chemotherapeutic compounds, anti-inflammatory compounds, antiviral compounds and/or immuno-modulating compounds.

The modified mixed sequence oligonucleotide or siRNAs of the invention can be used "as is" or in form of a variety of pharmaceutically acceptable salts. As used herein, the term "pharmaceutically acceptable salts" refers to salts that retain the desired biological activity of the oligonucleotide and exhibit minimal undesired toxicological effects. Non-limiting examples of such salts can be formed with organic amino acid and base addition salts formed with metal cations such as zinc, calcium, bismuth, barium, magnesium, aluminum, copper, cobalt, nickel, cadmium, sodium, potassium, and the like, or with a cation formed from ammonia, *N,N*-dibenzylethylene-diamine, D-glucosamine, tetraethylammonium, or ethylenediamine.

In one embodiment of the invention the mixed sequence oligonucleotide or modified siRNA may be in the form of a pro-drug. Suitable pro-drug formulations are described in PCT/DK2006/000512 and US provisional application 60/762,920.

Pharmaceutically acceptable binding agents and adjuvants may comprise part of the formulated drug, such as the binding agents and adjuvants described in PCT/DK2006/000512 and US provisional application 60/762,920.

The pharmaceutical compositions of the present invention may be administered in a number of ways depending upon whether local or systemic treatment is desired and upon the area to be treated. Suitable administration routes are described in PCT/DK2006/000512 and US provisional application 60/762,920.

Pharmaceutical compositions of the present invention include, but are not limited to, solutions, emulsions, and liposome-containing formulations, such as the formulations described in PCT/DK2006/000512 and US provisional application 60/762,920.

In another embodiment, compositions of the invention may contain one or more oligonucleotides according to the invention which are targeted to a first nucleic acid and one or more additional oligonucleotide compound, which may or may not be as according to the invention, which are targeted to a second nucleic acid target. Two or more combined compounds may be used together or sequentially.

The compounds disclosed herein are useful for a number of therapeutic applications as indicated above and those disclosed in PCT/DK2006/000512 and US provisional application 60/762,920. In general, therapeutic methods of the invention include administration of a therapeutically effective amount of a mixed sequence oligonucleotide or modified siRNA to a mammal, particularly a human. In a certain embodiment, the present invention provides pharmaceutical compositions containing (a) one or more compounds of the invention, and (b) one or more chemotherapeutic agents. When used with the compounds of the invention, such chemotherapeutic agents may be used individually, sequentially, or in combination with one or more other such chemotherapeutic agents or in combination with radiotherapy. Suitable chemotherapeutic agents are disclosed in PCT/DK2006/000512 and WO 2006/050734. Other active agents, such as anti-inflammatory drugs, including but not limited to nonsteroidal anti-inflammatory drugs and corticosteroids, antiviral drugs, and immuno-modulating drugs may also be combined in compositions of the invention. Two or more combined compounds may be used together or sequentially.

### Cancer

In an even further aspect the present invention relates to the use of mixed sequence oligonucleotide or a modified siRNA according to the invention for the manufacture of a medicament for the treatment of cancer. In another aspect the present invention concerns a method for treatment of, or prophylaxis against, cancer, said method comprising administering mixed sequence oligonucleotide or a modified siRNA of the invention or a pharmaceutical composition of the invention to a patient in need thereof.

PCT/DK2006/000512 and US provisional application 60/762,920 provide examples of cancers, which may also be treated by the pharmaceutical compositions of the present invention.

Similarly, the invention is further directed to the use of a mixed sequence oligonucleotide or a double stranded oligonucleotide according to the invention for the manufacture of a medicament for the treatment of cancer, wherein said treatment further comprises the administration of a further chemotherapeutic agent, such as the chemotherapeutic agents disclosed in PCT/DK2006/000512, WO 2006/050734. and US provisional application 60/762,920

Alternatively stated, the invention is furthermore directed to a method for treating cancer, said method comprising administering a double stranded oligonucleotide (e.g. modified siRNA) of the invention or a pharmaceutical composition according to the invention to a patient in need thereof and further comprising the administration of a further chemotherapeutic agent. Said further administration may be such that the further chemotherapeutic agent is conjugated to the compound of the invention, is present in the pharmaceutical composition, or is administered in a separate formulation.

### Infectious Diseases

It is contemplated that the compounds of the invention may be broadly applicable to a broad range of infectious diseases, such as diphtheria, tetanus, pertussis, polio, hepatitis B, hepatitis C, hemophilus influenza, measles, mumps, and rubella.

Accordingly, in yet another aspect the present invention relates the use of a mixed sequence oligonucleotide or a double stranded oligonucleotide (modified siRNA) according to the invention for the manufacture of a medicament for the treatment of an infectious disease, as well as to a method for treating an infectious disease, said method comprising administering a mixed sequence oligonucleotide or a modified siRNA according to the invention or a pharmaceutical composition according to the invention to a patient in need thereof.

### Inflammatory Diseases

In yet another aspect, the present invention relates to the use of a modified siRNA according to the invention for the manufacture of a medicament for the treatment of an inflammatory disease, as well as to a method for treating an inflammatory disease, said method comprising administering a modified siRNA according to the invention or a pharmaceutical composition according to the invention to a patient in need thereof.

In one preferred embodiment of the invention, the inflammatory disease is a rheumatic disease and/or a connective tissue diseases, such as rheumatoid arthritis, systemic lupus erythematous (SLE) or Lupus, scleroderma, polymyositis, inflammatory bowel disease, dermatomyositis, ulcerative colitis, Crohn's disease, vasculitis, psoriatic arthritis, exfoliative psoriatic dermatitis, pemphigus vulgaris and Sjorgren's syndrome, in particular inflammatory bowel disease and Crohn's disease.

Alternatively, the inflammatory disease may be a non-rheumatic inflammation, like bursitis, synovitis, capsulitis, tendinitis and/or other inflammatory lesions of traumatic and/or sportive origin.

### Metabolic diseases

In yet another aspect, the present invention relates to the use of a modified siRNA according to the invention for the manufacture of a medicament for the treatment of a metabolic disease, as well as to a method for treating a metabolic disease, said method comprising administering a modified siRNA according to the invention or a pharmaceutical composition according to the invention to a patient in need thereof.

In one preferred embodiment of the invention, the metabolic disease is selected form the group consisting of, diabetes, hyperlipidemia, hypercholesterolemia, and hyperlipoproteinema.

### Other Uses

The oligonucleotide of the invention may, in one embodiment target mammalian, such as human, Hif-1aplha mRNA. See WO 2006/050734, which refers to antisense oligonucleotides for down-regulation of Hif-1alpha. Suitably the oligonucleotide of the invention may consist or comprise any one of the sequences disclosed herein, and/or their complements, both in terms of the specific molecules disclosed in the sequence listings, and/or in terms of oligonucleotides which retain the sequence of nucleobases, but incorporate one or more of the features of the mixed sequence oligonucleotide or double stranded oligonucleotides as referred to herein. Hif-1alpha oligonucleotides may be used in the treatment of numerous diseases such as cancer, atherosclerosis, psoriasis, diabetic retinopathy, rheumatoid arthritis, asthma, or inflammatory bowel disease. It is envisaged that the oligonucleotides of the invention, may, in one embodiment comprise one or two mismtaches to the Hlf-1alpha target mRNA.

The mixed sequence oligonucleotide or the modified siRNAs of the present invention can be utilized for as research reagents for diagnostics, therapeutics and prophylaxis. In research, the mixed sequence oligonucleotide or the modified siRNA may be used to specifically inhibit the synthesis of target genes in cells and experimental animals thereby facilitating functional analysis of the target or an appraisal of its usefulness as a target for therapeutic intervention. In diagnostics the mixed sequence oligonucleotide or the siRNA oligonucleotides may be used to detect and quantitate target expression in cell and tissues by Northern blotting, *in-situ* hybridisation or similar techniques. For therapeutics, an animal or a human, suspected of having a disease or disorder, which can be treated by modulating the expression of target is treated by administering the mixed sequence oligonucleotide or the modified siRNA compounds in accordance with this invention. Further provided are methods of treating an animal particular mouse and rat and treating a human, suspected of having or being prone to a disease or condition, associated with expression of target by administering a therapeutically or prophylactically effective amount of one or more of the mixed sequence oligonucleotide or the modified siRNA compounds or compositions of the invention.

The invention is further illustrated in a non-limiting manner by the following examples.

### EXAMPLES

### Abbreviations

| | |
|---|---|
| DMT: | Dimethoxytrityl |
| DCI: | 5-Dicyanoimidazole |
| DMAP: | 4-Dimethylaminopyridine |
| DCM: | Dichloromethane |
| DMF: | Dimethylformamide |
| THF: | Tetrahydrofuran |
| DIEA: | *N*,*N*-diisopropylethylamine |
| Py BOP: | Benzotriazole-1-yl-oxy-tris pyrrolidino-phosphonium hexafluorophosphate |
| Bz: | Benzoyl |
| Ibu. | Isobutyryl |
| Beaucage: | 3H-1,2-Benzodithiole-3-one-1,1-dioxide |

### Sequences:

siRNA/siLNA sequence:
   5'- ccu acu gca ggg uga aga a dtdt- 3' (sense) (SEQ ID NO 1)
   3'- dtdt gga uga cgu ccc acu ucu u- 5 ' (antisense) (SEQ ID NO 2)
**LNA sense strand versions (P=O backbone and P=S backbone):**
   5'- **C**cu acu gca ggg uga aga a **TT**- 3' (sense) (SPC 3175 P=O; SPC3178 P=S) (SEQ ID NO 3 & 4)
   5'- **C**cu acu g**C**a **G**gg uga aga a **TT**- 3' (sense) (SPC 3177 P=O; SPC3176 P=S) (SEQ ID NO 5 & 6)
   5'- **C**cu acu g**C**a Ggg u**G**a aga a **TT**- 3' (sense) (SPC 3179 P=O; SPC3180 P=S) (SEQ ID NO 7 & 8)
   5'- ccu acu gca ggg uga aga a dtdt- 3' (sense) (SPC 3181 P=O; SPC3182 P=S) (SEQ ID NO 9 & 10)
**LNA sense strand versions (P=S backbone):**
   5 '-**C**cₛuₛ aₛcₛuₛ gₛ**C**aₛ **G**gₛgₛ uₛgₛaₛ aₛgₛaₛ aₛ **TT**- 3 ' (SPC3347) (SEQ ID NO 11)
   5'-**C**cₛuₛ aₛcₛuₛ gₛ**C**aₛ **G**ₛgₛgₛ uₛgₛaₛ aₛgₛaₛ as **TT**- 3' (SPC3389)(For "native Tₘ) (SEQ ID NO 12)
**LNA sense strand versions (P=O backbone):**
   5' - Ccu acu gCa Gₛgg uga aga a **TT**- 3' (SPC3391) (For "native Tₘ) (SEQ ID NO 13)
**LNA antisense strand versions (P=O backbone and P=S backbone):**
   5'- uuc uuc acc cug cag uag g **TT**- 3' (antisense) (SPC 3183 P=O; SPC3184 P=S) (SEQ ID NO 14 & 15)
   5'- uuc uuc acc cug cag uag g dtdt- 3' (antisense) (SPC 3185 P=S; SPC3186 P=O) (SEQ ID NO 16 & 17)
   bold uppercase: Beta-D-oxy LNA monomer, lowercase: RNA, dt: deoxythymidine, subscript "s": thiolated diesesterbond (otherwise full phosphodiester or phosphorothioaltion).

### Example 1: Monomer Synthesis

The preparation of LNA monomers is described in great detail in the references Koshkin et al., J.Org.Chem., 2001,66,8504-8512, and Pedersen et al., Synthesis, 2002,6,802-809 as well as in references given therein. Where the Z and Z* protection groups were oxy-N,N-diisopropyl-O-(2-cyanoethyl)phosphoramidite and dimethoxytrityloxy such compounds were synthesised as described in WO 03/095467; Pedersen et al., Synthesis 6, 802-808, 2002; Sørensen et al., J. Am. Chem. Soc., 124, 2164-2176, 2002; Singh et al., J. Org. Chem. 63, 6078-6079, 1998; and Rosenbohm et al., Org. Biomol. Chem. 1, 655-663, 2003. All cytosine-containing monomers were replaced with 5-methyl-cytosine monomers for all couplings. All LNA monomers used were beta-D-oxy LNA (compound **2A**).

### Example 2: Oligonucleotide Synthesis

All syntheses were carried out in 1 µmole scale on a MOSS Expedite instrument platform. The synthesis procedures were carried out essentially as described in the instrument manual.

### Preparation of LNA Succinyl Hemiester

5'-O-DMT-3"hydroxy-LNA monomer (500 mg), succinic anhydride (1.2 eq.) and DMAP (1.2 eq.) were dissolved In DCM (35 ml). The reaction mixture was stirred at room temperature overnight. After extraction with NaH₂PO₄, 0.1 M, pH 5.5 (2x), and brine (1x), the organic layer was further dried with anhydrous Na₂SO₄, filtered, and evaporated. The hemiester derivative was obtained in a 95% yield and was used without any further purification.

### Preparation of LNA-CPG (Controlled Pore Glass)

The above-prepared hemiester derivative (90 µmole) was dissolved in a minimum amount of DMF. DIEA and pyBOP (90 µmole) were added and mixed together for 1 min. This preactivated mixture was combined with LCAA-CPG (500 Å, 80-120 mesh size, 300 mg) in a manual synthesiser and stirred. After 1.5 h stirring at room temperature, the support was filtered off and washed with DMF, DCM and MeOH. After drying the loading was determined to be 57 µmol/g (see Tom Brown, Dorcas J.S.Brown. Modern machine-aided methods of oligodeoxyribonucleotide synthesis. In: F.Eckstein, editor. Oligonucleotides and Analogues A Practical Approach. Oxford: IRL Press, 1991: 13-14).

### Phosphorothioate Cycles

5'-O-DMT (A(bz), C(bz), G(ibu) or T) linked to CPG were deprotected using a solution of 3% trichloroacetic acid (v/v) in dichloromethane. The CPG was washed with acetonitrile. Coupling of phosphoramidites (A(bz), G(ibu), 5-methyl-C(bz)) or T-β-cyanoethyl-phosphoramidite) was performed by using 0.08 M solution of the 5'-O-DMT-protected amidite in acetonitrile and activation was done by using DCI (4,5-dicyanoimidazole) in acetonitrile (0.25 M). The coupling reaction was carried out for 2 min. Thiolation was carried out by using Beaucage reagent (0.05 M in acetonitrile) and was allowed to react for 3 min. The support was thoroughly washed with acetonitrile and the subsequent capping was carried out by using standard solutions (CAP A) and (CAP B) to cap unreacted 5' hydroxyl groups. The capping step was then repeated and the cycle was concluded by acetonitrile washing.

### LNA Unit Cycles

5'-O-DMT (A(bz), C(bz), G(ibu) or T) linked to CPG was deprotected by using the same procedure as described above. Coupling was performed by using 5'-O-DMT-A(bz), C(bz), G(ibu) or T-β-cyanoethylphosphoramidite (0.1 M in acetonitrile) and activation was done by DCI (0.25 M in acetonitrile). The coupling reaction was carried out for 7 minutes. Capping was done by using standard solutions (CAP A) and (CAP B) for 30 sec. The phosphite triester was oxidized to the more stable phosphate triester by using a standard solution of I₂ and pyridine in THF for 30 sec. The support was washed with acetonitrile and the capping step was repeated. The cycle was concluded by thorough acetonitrile wash.

### Cleavage and Deprotection

The oligonucleotides were cleaved from the support and the β-cyanoethyl protecting group removed by treating the support with 35% NH₄OH for 1 h at room temperature. The support was filtered off and the base protecting groups were removed by raising the temperature to 65°C for 4 hours. Ammonia was then removed by evaporation.

### Purification

The oligos were either purified by reversed-phase-HPLC (RP-HPLC) or by anion exchange chromatography (AIE):

| *RP-HPLC:* | |
|---|---|
| Column: | VYDAC^{™}, Cat. No. 218TP1010 (vydac) |
| Flow rate: | 3 ml/min |
| Buffer: | A (0.1 M ammonium acetate, pH 7.6) |
| | B (acetonitrile) |

| *Gradient:* | | | | | | |
|---|---|---|---|---|---|---|
| Time | 0 | 10 | 18 | 22 | 23 | 28 |
| B% | 0 | 5 | 30 | 100 | 100 | 0 |

| *AIE:* | |
|---|---|
| Column: | Resource^{™} 15Q (amersham pharmacia biotech) |
| Flow rate: | 1.2 ml/min |
| Buffer: | A (0.1 M NaOH) |
| | B (0.1 M NaOH, 2.0 M NaCl) |

| *Gradient:* | | | | | | |
|---|---|---|---|---|---|---|
| Time | 0 | 1 | 27 | 28 | 32 | 33 |
| 8% | 0 | 25 | 55 | 100 | 100 | 0 |

### Tₘ Measurement

100 µl 15 µM siRNA/siLNA duplex stock in H₂O was diluted with 400 µl H₂O, where after 500 µl 2x Tm-buffer (200 mM NaCl, 0,2 mM EDTA, 20 mM NaP, pH 7,0 , this buffer was also DEPC treated to remove RNases) was also added (final duplex conc 1,5 µM). The solution was heated to 95°C for 3 min and then allowed to anneal in RT for 30 min.

Tₘ was measured on Lambda 40 UV/VIS Spectrophotometer with peltier temperature progammer PTP6 using PE Templab software (Perkin Elmer). The Temperature was ramped up from 20°C to 95°C and then down again to 25°C, recording absorption at 260 nm. First derivative and local maximums of both the melting and annealing was used to assess melting/annealing point, both that should give similar/same Tₘ values. For the first derivative 91 points (maximum) was used to calculate the slope, this to get a smooth derivative curve for all duplexes so they were all treated equally.

### Example 3: Synthesis of LNA/RN Oligonucleotides

### Synthesis

LNA/RNA oligonucleotides were synthesized DMT-off on a 1.0 µmole scale using an automated nucleic acid synthesiser (MOSS Expedite 8909) and using standard reagents. 1*H*-tetrazole or 5-ethylthio-1*H*-tetrazole were used as activators. The LNA A^{Bz}, G^{iBu} and T phosphoramidite concentration was 0.1 M in anhydrous acetonitrile. The ^{Me}C^{Bz} was dissolved in 15 % THF in acetonitrile. The coupling time for all monomer couplings was 600 secs. The RNA phosphoramidites (Glen Research, Sterling, Virginia) were *N*-acetyl and 2'-O-triisopropylsilyloxymethyl (TOM) protected. The monomer concentration was 0.1 M (anhydrous acetonitrile) and the coupling time was 900 secs. The oxidation time was set to be 50 sec. The solid support was DMT-LNA-CPG (1000 Å, 30-40 µmole/g).

### Work-up and Purification

Cleavage from the resin and nucleobase/phosphate deprotection was carried out in a sterile tube by treatment with 1.5 ml of a methylamine solution (1:1, 33% methylamine in ethanol:40% methylamine in water) at 35°C for 6 h or left overnight. The tube was centrifuged and the methylamine solution was transferred to second sterile tube. The methylamine solution was evaporated in a vacuum centrifuge. To remove the 2'-*O-*protection groups the residue was dissolved in 1.0 ml 1.0 M TBAF in THF and heated to 55°C for 15 min. and left at 35°C overnight. The THF was evaporated in a vacuum centrifuge leaving a light yellow gum, which was neutralised with approx. 600 µl (total sample volume: 1.0 ml) of RNase-free 1.0 M Tris-buffer (pH 7). The mixture was homogenised by shaking and heating to 65°C for 3 min. Desalting of the oligonucleotides was performed on NAP-10 columns (Amersham Biosciences, see below). The filtrate from step 4 (see below) was collected and analysed by MALDI-TOF and gel electroforesis (16% sequencing acrylamide gel (1 mm), 0.9% TBE [Tris: 89 mM, Boric acid: 89 mM, EDTA: 2 mM, pH 8.3] buffer, ran for 2 h at 20 W as the limiting parameter. The gel was stained in CyberGold (Molecular Probes, 1:10000 in 0.9xTBE) for 30 min followed by scanning in a Bio-Rad FX Imager). The concentration of the oligonucleotide was measured by UV-spectrometry at 260 nm.

### Scheme A, Desalting on NAP-10 columns:

| Step | Reagent | Operation | Volume | Remarks |
|---|---|---|---|---|
| 1 | - | Empty storage buffer | - | Discard |
| 2 | H₂O (RNase-free) | Wash | 2 x full volume | Discard |
| 3 | Oligo in buffer (RNase-free) | Load | 1.0 ml | Discard |
| 4 | H₂O (RNase-free) | Elution | 1.5 ml | Collect - Contains oligo |
| 5 | H₂O (RNase-free) | "Elution" | 0.5 ml | Collect - Contains salt + small amount of oligo |

As will be appreciated by the skilled person, the most important issues in the synthesis of the LNA/RNA oligos as compared to standard procedures are that i) extended coupling times are necessary to achieve good coupling efficiency, and ii) the oxidation time has to be extended to minimise the formation of deletion fragments. Furthermore, coupling of 2'-O-TOM protected phosphoramidites were superior to 2'-*O*-TBDMS. Taking this into account, the crude oligonucleotides were of such quality that further purification could be avoided. MS analysis should be carried out after the TOM-groups are removed.

### Example 4: Test of Design of modified siRNA in Mammalian System

### HIF-1A MRNA ASSAY

The different siLNA were transfected in cell culture (BNL CL.2, mouse liver) at 1, 10 and 100 nM. Hif-1a mRNA levels were measured by qPCR. The RNA antagonist SPC2968, an LNA and phosphorothiolated singlestranded antisense oligonucleotide, with verified effect on Hif-1a both in vitro and in vivo, served as positive control. Shown is Hif-1a mRNA levels from experiments as percentage of mock transfected cells. Shown are also schematics of the siLNA and identification number.

### Example 5 LNA can increase or decrease Tₘ depending on environment

RNA/LNA containing oligonucleotides were synthesized, all having the same sequence or complementary sequence, containing either phosphodiester or phosphorothloate linkage. Different duplex combinations were created by hybridizing differently modified oligonucleotides to its complementary counter part. Melting temeprature (Tₘ) was measured for the different duplex combinations.

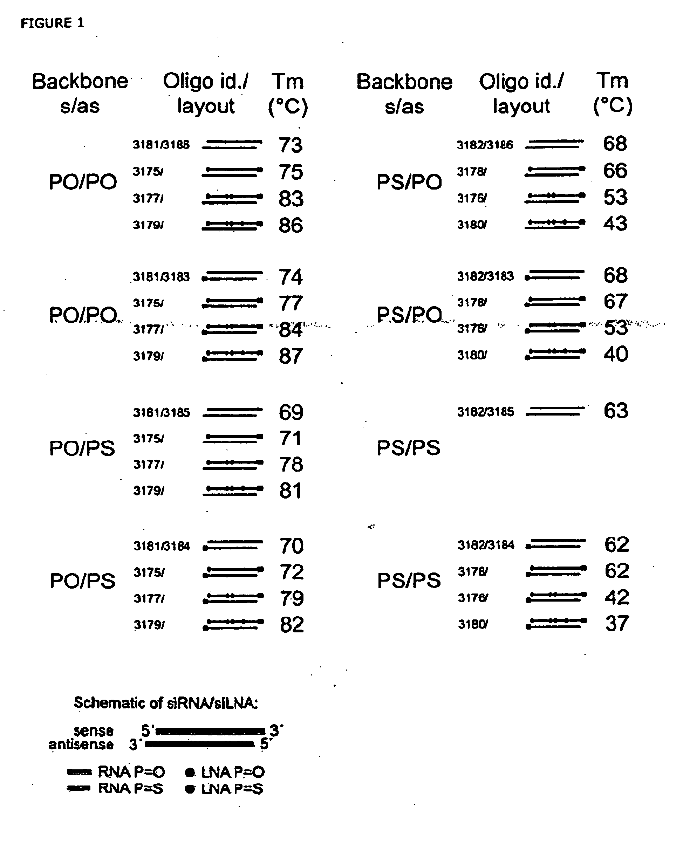

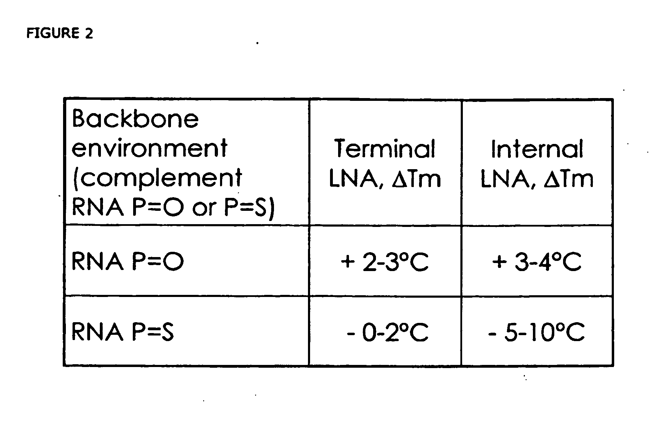

LNA in a RNA, phosphodiester environment increase Tₘ whether the complementary strand is phosphorothiolated or not. LNA in a RNA, phosphotothioate environment reduce the Tₘ whether the complementary strand is phosphorothiolated or not (Figure 1). The estimated increase or decrease per LNA base depending on surrounding is summarized in Figure 2.

### Example 6 Specifically the linkage 3' to LNA in LNA/RNA oligonucleotide modulates Tₘ

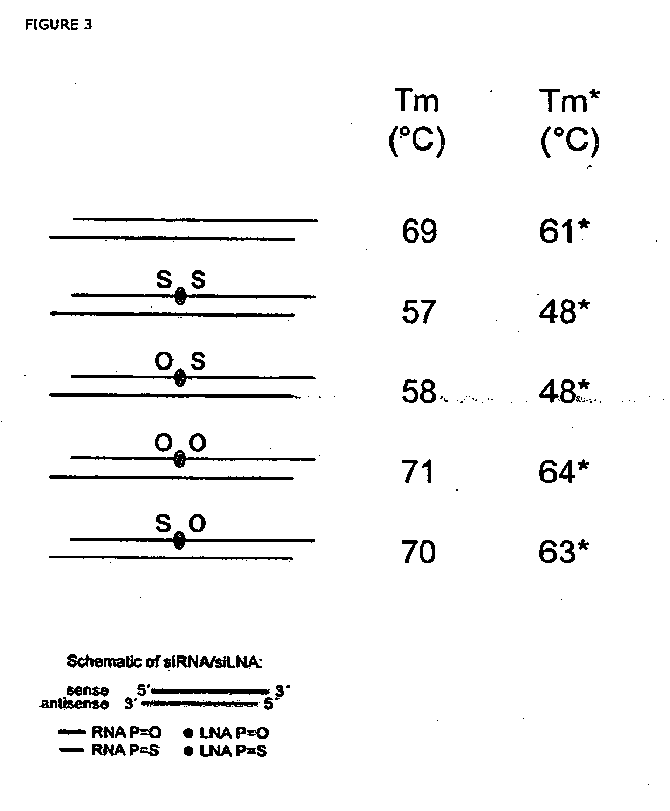

RNA/LNA oligonucleotides were synthesized with only one LNA in the central position, with fully phosphorothiolated linkage. Different combinations of reverting the phosphorothioate to phosphorodiester at the linkage 5', 3' or both to the LNA were also synthesized. These oligonucleotides were combined with either a full RNA or DNA compementary strand and Tₘ was measured. Specifically the phosphorythiolation in the 3' position to LNA decreases Tₘ, whereas phosphodiester 3' to the LNA increase Tₘ. (see Figure 3)

Phosphodiester bond 3' to LNA in an otherwise phosphorothioate environment increases Tₘ.

A fully phosphorythiolated oligonucleotide containing LNA were compared with the same oligonucleotide with phosphodiester bond in the 3 position to the LNAs for its hybridization properties to its complementary strand by measuring Tₘ.

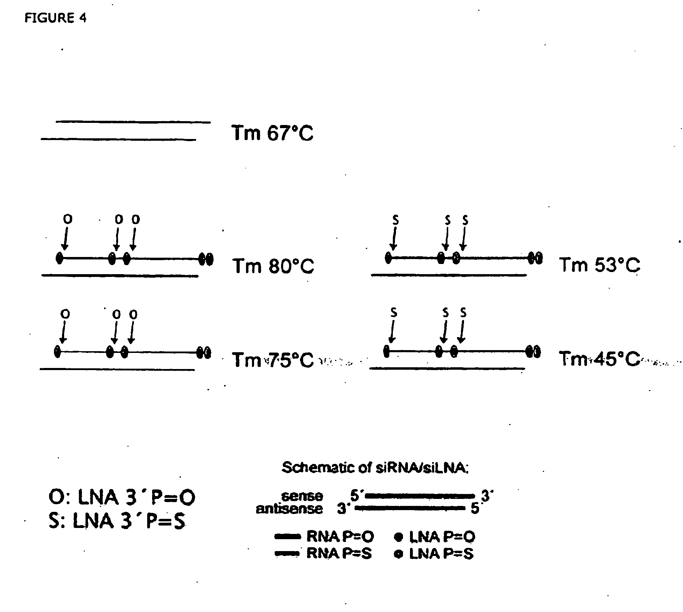

Replacement of the phosphrothioate with phosphodiester 3' to the LNA increase Tₘ. (see Figure 4)

### Example 7 Phosphorathioate bond 3' to LNA in an otherwise phosphoradiester environment reduces Tₘ.

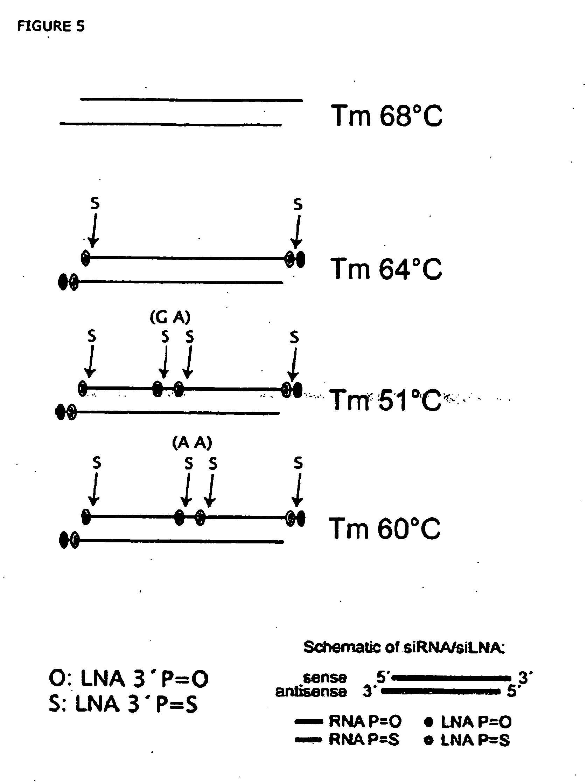

Tₘ was measured on oligonucleotides with phosphorothioate linked only in the 3' position to the LNA modifications. Tₘ could still be reduced even though most of the oligonucleotide contained phosphodiester bonds (Figure 5).

### Example 8 LNA enhances nuclease stability in both phosphorodiester and phosphorothioate compounds

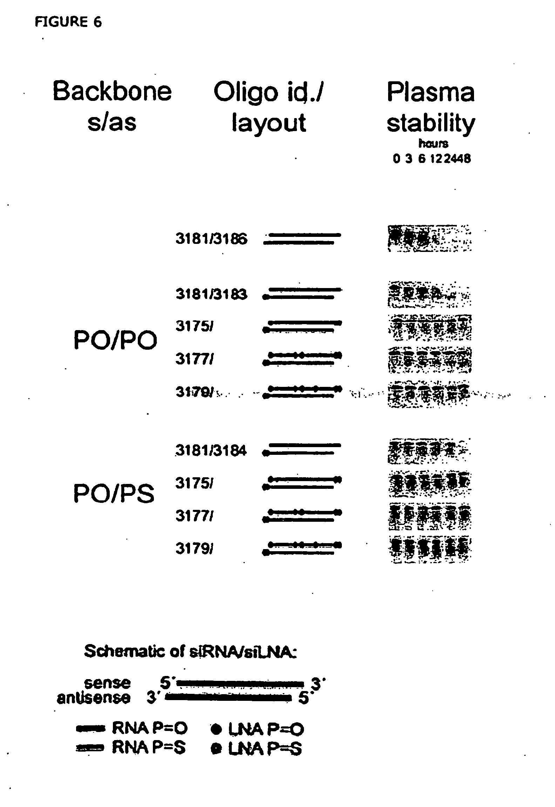

Some of the previously mentioned LNA/RNA/PS/PO duplexes were tested for their nuclease stability by incubation in mouse serum at 37°C, phenolextraced and analyzed by native PAGE. LNA enhances the serum stability and the phosphorothioate modification alone appears also have some contribution to nuclease resistance (Figure 6).

### Example 9 Too high Tₘ reduces effect. LNA/RNA/PS/PO duplexes have gene-silencing effect on target mRNA. Too low Tₘ reduces effect

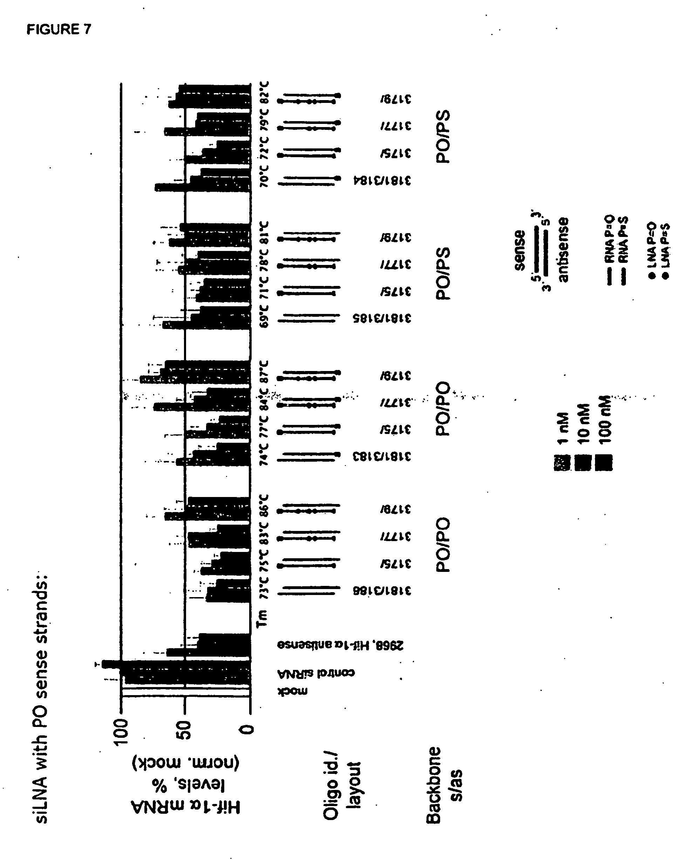

The previously mentioned LNA/RNA/PS/PO duplexes were tested for their ability to inhibit target mRNA in cell culture. The duplexes were transfected at three concentrations (1, 10, 100 nM) into BNL CL.2 mouse fibroblasts and incubated for 24 hours, where after the cells were harvested and RNA extracted. The target RNA was quantified using quantitative PCR (qPCR). Several combinations showed inhibitory effect (Figure 7)

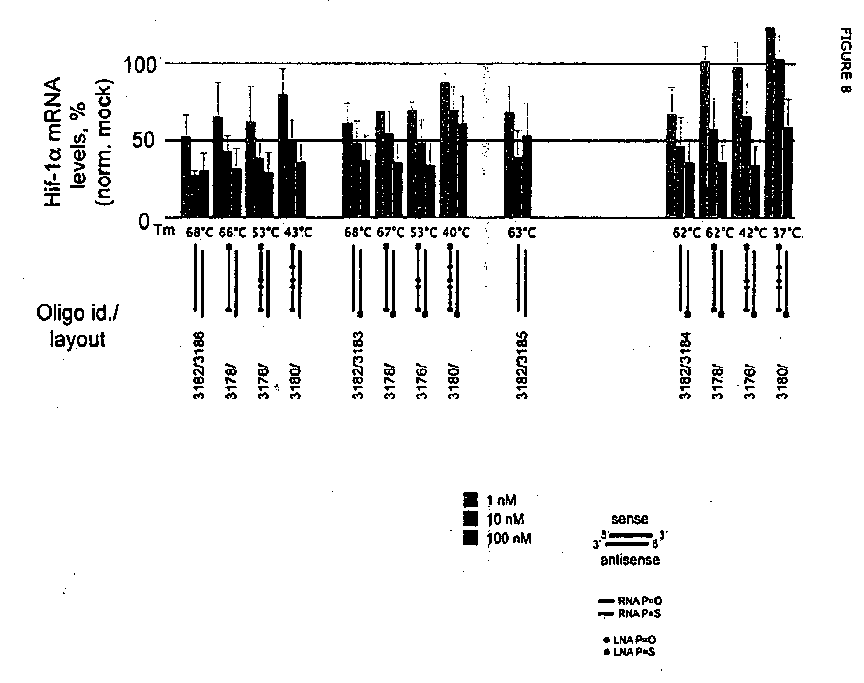

Also, a too low Tₘ reduces the effect (See Figure 8)

### Example 10 Optimizing TM for good effect

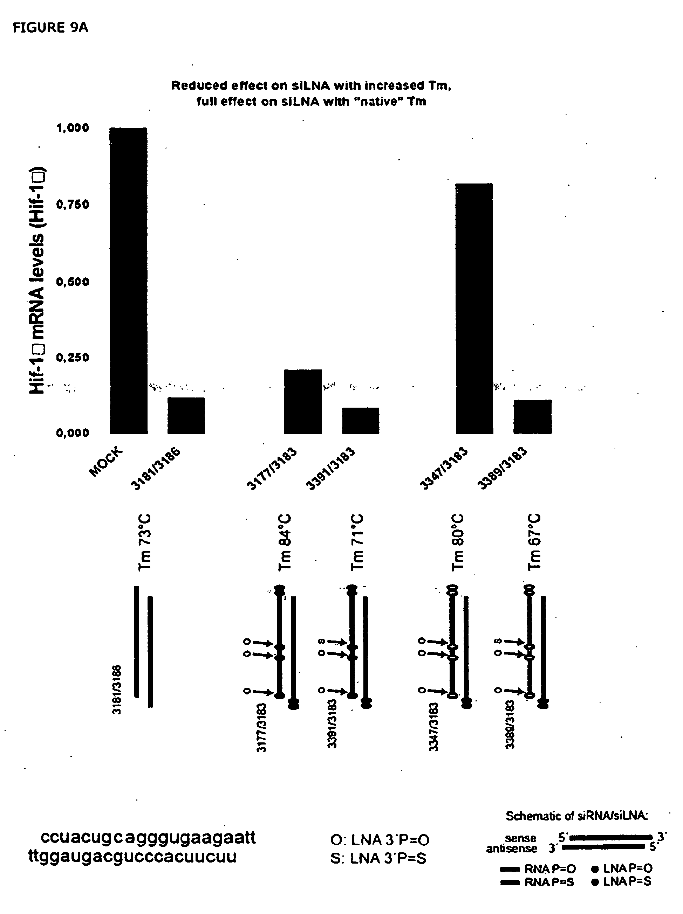

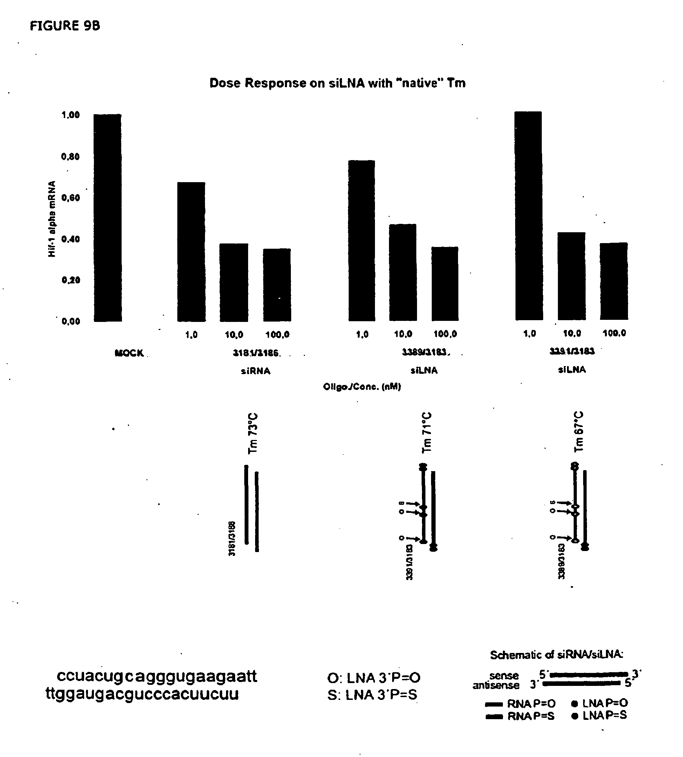

Nuclease protected LNA modified siRNA have high Tₘ (compound 3347/3183 and 3177/3182) and display an reduced inhibitory capacity (Figure 9a). Keeping the amount of LNA constant for nuclease protection but lowering Tₘ by a PS bond 3 ' an LNA modulates Tₘ (compound 3391/8183 and 3389/3183) to an "native" state, similar to unmodified (not nuclease protected) siRNA. However, such Tₘ optimised constructs have full inhibitory effect as compared to the unmodified siRNA. siLNA with "native" Tₘ have been compared to unmodified siRNA in a dosis-response study which show equal inhibitory effect on siRNA and siLNA having similar Tₘ (Figure 9B).

## Claims

1. A double stranded oligonucleotide for RNAi, comprising between 15-25 nucleotides in each strand, wherein at least one of the two stands comprises a mixed sequence oligonucleotide comprising at least one dinucleotide of sequence 5' LNA-PS-XNA 3', wherein; XNA is either an RNA nucleotide or an RNA nucleotide analogue which comprises a 2' substitution, wherein the 2' substitution is other than hydrogen; LNA is a locked nucleic acid; and PS is a phosphorothioate internucloside linkage -O-P(O,S)-O-, and; wherein the mixed sequence oligonucleotide comprises both phosphorothioate and phosphodiester linkages.

2. The double stranded oligonucleotide according to claim 1, wherein the remaining nucleobases of the mixed sequence oligonucleotide are independently selected from the group consisting of LNA and RNA nucleobases.

3. The double stranded oligonucleotide according to claim 2, wherein both the first (e.g. sense) strand and the second (e.g. antisense) strand comprises both 3'-overhangs.

4. The double stranded oligonucleotide according to claim 1, wherein the mixed sequence oligonucleotide comprises 1 - 4 5'LNA-PS-XNA-3' dinucleotide sequences.

5. The double stranded oligonucleotide according to claim 3, wherein said 3'-overhang is between 1 - 3 monomers in length.

6. The double stranded oligonucleotide according to any one of claims 1 - 5, wherein each strand consists of between 17 - 22 nucleobases in length.

7. The double stranded oligonucleotide according to any one of claims 1 - 6, wherein the XNA nucleotide of the dinucleotide sequence is an RNA unit.

8. The double stranded oligonucleotide according to any one of claims 1, or 3 - 6, wherein the XNA nucleotide of the dinucleotide sequence is an RNA nucleotide analogue, which comprises said 2' substitution.

9. The double stranded oligonucleotide according to claim 8, wherein in the RNA analogue comprises a 2' fluoro group, in either arabino- or ribo- configuration or wherein said 2' substitution is a 2' O substituent, such as a 2' O substituent selected from the group consisting of 2'O alkyl, 2'O methyl, 2'Omethoxyethyl.

10. The double stranded oligonucleotide according to any one of claims 1 - 9, wherein the LNA nucleotide is selected from the group consisting of thio-LNA, amino-LNA, oxy-LNA, and ena-LNA in either the beta-D or alpha-L configurations.

11. The double stranded oligonucleotide according to any one of claims 1 - 10, wherein the all the remaining internucleoside linkages are phosphodiester linkages.

12. The double stranded oligonucleotide according to any one of claims 1-11, for use as a siRNA.

13. The double stranded oligonucleotide according to any one of claims 1 - 12, wherein the first strand, such as the sense strand of the siRNA, comprises 1 or 2 5'LNA monomers and 1 or 2 3'LNA momomers.

14. The double stranded oligonucleotide according to any one of claims 1 - 13, wherein the second strand, such as the antisense strand of the siRNA, comprises 1 or 2 3' LNA monomers, and no LNA monomer is located within 1 nucleotide of the 5'end.

15. The double stranded oligonucleotide according to any one of claims 1 - 12, wherein the first strand comprises 1 or 2 5'LNA monomers and 1 or 2 3'LNA momomers, and wherein the second strand comprises 1 or 2 3' LNA monomers and wherein no LNA monomer is located at the 5'end of second strand.

## Patentansprüche

1. Doppelsträngiges Oligonukleotid für RNAi, umfassend 15-25 Nukleotide in jedem Strang, wobei mindestens einer der zwei Stränge ein Mischsequenzoligonukleotid umfasst, umfassend mindestens ein Dinukleotid mit der Sequenz 5'-LNA-PS-XNA-3', wobei XNA entweder ein RNA-Nukleotid oder ein RNA-Nukleotidanalogon ist, das eine 2'-Substitution umfasst, wobei die 2'-Substitution von Wasserstoff verschieden ist; LNA eine Locked-Nucleic-Acid ist; und PS eine Phosphorthioat-Internukleosid-Bindung OP(O,S)-O- ist; und wobei das Mischsequenzoligonukleotid sowohl Phosphorthioat- als auch Phosphodiesterbindungen aufweist.

2. Doppelsträngiges Oligonukleotid nach Anspruch 1, wobei die restlichen Nukleobasen des Mischsequenzoligonukleotids unabhängig voneinander ausgewählt sind aus der Gruppe, bestehend aus LNA- und RNA-Nukleobasen.

3. Doppelsträngiges Oligonukleotid nach Anspruch 2, wobei sowohl der erste (z. B. sense) Strang als auch der zweite (z. B. antisense) Strang beide 3'-Überhänge umfassen.

4. Doppelsträngiges Oligonukleotid nach Anspruch 1, wobei das Mischsequenzoligonukleotid 1-4 5'-LNA-PS-XNA-3'-Dinukleotidsequenzen umfasst.

5. Doppelsträngiges Oligonukleotid nach Anspruch 3, wobei der 3'-Überhang eine Länge von 1 bis 3 Monomeren aufweist.

6. Doppelsträngiges Oligonukleotid nach einem der Ansprüche 1-5, wobei jeder Strang eine Länge zwischen 17 und 22 Nukleobasen aufweist.

7. Doppelsträngiges Oligonukleotid nach einem der Ansprüche 1-6, wobei das XNA-Nukleotid der Dinukleotidsequenz eine RNA-Einheit ist.

8. Doppelsträngiges Oligonukleotid nach einem der Ansprüche 1 oder 3-6, wobei das XNA-Nukleotid der Dinukleotidsequenz ein RNA-Nukleotidanalogon ist, das die 2'-Substitution umfasst.

9. Doppelsträngiges Oligonukleotid nach Anspruch 8, wobei das RNA-Analogon eine 2'-Fluorgruppe entweder in der Arabin- oder der Ribokonfiguration umfasst oder wobei die 2'-Substitution ein 2'-O-Substituent ist, wie ein 2'-O-Substituent, ausgewählt aus der Gruppe, bestehend aus 2'-O-Alkyl, 2'-O-Methyl, 2'-O-Methoxyethyl.

10. Doppelsträngiges Oligonukleotid nach einem der Ansprüche 1-9, wobei das LNA-Nukleotid ausgewählt ist aus der Gruppe, bestehend aus Thio-LNA, Amino-LNA, Oxy-LNA und Ena-LNA in entweder der beta-D- oder der alpha-L-Konfiguration.

11. Doppelsträngiges Oligonukleotid nach einem der Ansprüche 1-10, wobei alle die restlichen Internukleosidbindungen Phosphodiesterbindungen sind.

12. Doppelsträngiges Oligonukleotid nach einem der Ansprüche 1-11 zur Verwendung als eine siRNA.

13. Doppelsträngiges Oligonukleotid nach einem der Ansprüche 1-12, wobei der erste Strang, wie der sense-Strang der siRNA, 1 oder 2 5'-LNA-Monomere und 1 oder 2 3'-LNA-Monomere umfasst.

14. Doppelsträngiges Oligonukleotid nach einem der Ansprüche 1-13, wobei der zweite Strang, wie der antisense-Strang der siRNA, 1 oder 2 3'-LNA-Monomere umfasst und sich am 5'-Ende kein LNA-Monomer im ersten Nukleotid befindet.

15. Doppelsträngiges Oligonukleotid nach einem der Ansprüche 1-12, wobei der erste Strang 1 oder 2 5'-LNA-Monomere und 1 oder 2 3'-LNA-Monomere umfasst und wobei der zweite Strang 1 oder 2 3'-LNA-Monomere umfasst und sich am 5'-Ende des zweiten Strangs kein LNA-Monomer befindet.

## Revendications

1. Oligonucléotide à double brin pour ARNi, comprenant 15 à 25 nucléotides dans chaque brin, dans lequel au moins l'un des deux brins comprend un oligonucléotide à séquence mixte comprenant au moins un dinucléotide de séquence 5' LNA-PS-XNA 3', où XNA est soit un nucléotide d'ARN soit un analogue de nucléotide d'ARN qui comprend une substitution en 2', où la substitution en 2' est autre que de l'hydrogène ; LNA est un acide nucléique verrouillé et PS est une liaison internucléosidique phosphorothioate -O-P(O,S)-O-, et dans lequel l'oligonucléotide à séquence mixte comprend à la fois des liaisons phosphorothioate et phosphodiester.

2. L'oligonucléotide à double brin selon la revendication 1, dans lequel les nucléobases restantes de l'oligonucléotide à séquence mixte sont choisies indépendamment dans le groupe consistant en des nucléobases de LNA et d'ARN.

3. L'oligonucléotide à double brin selon la revendication 2, dans lequel le premier brin (sens) et le deuxième brin (antisens) comprennent tous deux des extrémités sortantes en 3'.

4. L'oligonucléotide à double brin selon la revendication 1, dans lequel l'oligonucléotide à séquence mixte comprend des séquences dinucléotidiques 1 - 4 5'LNA-PS-XNA-3'.

5. L'oligonucléotide à double brin selon la revendication 3, dans lequel ladite extrémité sortante en 3' présente une longueur de 1 à 3 monomères.

6. L'oligonucléotide à double brin selon l'une quelconque des revendications 1 à 5, dans lequel chaque brin présente une longueur de 17 à 22 nucléobases.

7. L'oligonucléotide à double brin selon l'une quelconque des revendications 1 à 6, dans lequel le nucléotide XNA de la séquence dinucléotidique est une unité d'ARN.

8. L'oligonucléotide à double brin selon l'une quelconque des revendications 1 à 6, dans lequel le nucléotide XNA de la séquence dinucléotidique est un analogue de nucléotide d'ARN, qui comprend ladite substitution en 2'.

9. L'oligonucléotide à double brin selon la revendication 8, dans lequel l'analogue de l'ARN comprend un groupement fluoro en 2', dans une configuration arabino- ou ribo-, ou dans lequel ladite substitution en 2' est un substituant O en 2', tel qu'un substituant O en 2' choisi dans le groupe consistant en O alkyle en 2', O méthyle en 2', O méthoxyéthyle en 2'.

10. L'oligonucléotide à double brin selon l'une quelconque des revendications 1 à 9, dans lequel le nucléotide LNA est choisi dans le groupe consistant en thio-LNA, amino-LNA, oxy-LNA et ena-LNA en configurations bêta-D ou alpha-L.

11. L'oligonucléotide à double brin selon l'une quelconque des revendications 1 à 10, dans lequel toutes les liaisons internucléosidiques restantes sont des liaisons phosphodiester.

12. L'oligonucléotide à double brin selon l'une quelconque des revendications 1 à 11, destiné à être utilisé comme ARNsi.

13. L'oligonucléotide à double brin selon l'une quelconque des revendications 1 à 12, dans lequel le premier brin, tel que le brin sens de l'ARNsi, comprend 1 ou 2 monomères de LNA en 5' et 1 ou 2 monomères de LNA en 3'.

14. L'oligonucléotide à double brin selon l'une quelconque des revendications 1 à 13, dans lequel le deuxième brin, tel que le brin antisens de l'ARNsi, comprend 1 ou 2 monomères de LNA en 3' et aucun monomère de LNA ne se trouve dans un nucléotide de l'extrémité 5'.

15. L'oligonucléotide à double brin selon l'une quelconque des revendications 1 à 12, dans lequel le premier brin comprend 1 ou 2 monomères de LNA en 5' et 1 ou 2 monomères de LNA en 3', et dans lequel le deuxième brin comprend 1 ou 2 monomères de LNA en 3' et dans lequel aucun monomère de LNA ne se trouve à l'extrémité 5' du deuxième brin.


### SEQUENCE LISTING

<110> Santaris A/S
<120> LNA MODIFIED PHOSPHOROTHIOLATED OLIGONUCLEOTIDES
<130> 16834PCT00
<160> 17
<170> Patent In version 3-3
<210> 1
   <211> 21
   <212> DNA
   <213> Artificial
<220>
   <223> siRNA/siLNA sense seqeunce
<220>
   <221> RNA
   <222> (1)..(19)
<220>
   <221> deoxythymidine
   <222> (20)..(21)
<400> 1
   ccuacugcag ggugaagaat t 21
<210> 2
   <211> 21
   <212> DNA
   <213> Artificial
<220>
   <223> siRNA/siLNA antisense sequence
<220>
   <221> deoxythymidine
   <222> (1)..(2)
<220>
   <221> RNA
   <222> (1)..(19)
<400> 2
   ttggaugacg ucccacuucu u 21
<210> 3
   <211> 21
   <212> DNA
   <213> Artificial
<220>
   <223> LNA sense P=O backbone
<220>
   <221> Beta-D-oxy LNA monomer
   <222> (1)..(1)
<220>
   <221> P=O backbone
   <222> (1)..(21)
<220>
   <221> RNA
   <222> (2)..(19)
<220>
   <221> Beta-D-oxy LNA monomer
   <222> (20)..(21)
<400> 3
   ccuacugcag ggugaagaat t 21
<210> 4
   <211> 21
   <212> DNA
   <213> Artificial
<220>
   <223> LNA sense - P=S backbone
<220>
   <221> Beta-D-oxy LNA
   <222> (1)..(1)
<220>
   <221> P=S backbone
   <222> (1)..(21)
<220>
   <221> RNA
   <222> (2) .. (19)
<220>
   <221> Beta-D-oxy LNA
   <222> (20)..(21)
<400> 4
   ccuacugcag ggugaagaat t 21
<210> 5
   <211> 21
   <212> DNA
   <213> Artificial
<220>
   <223> siRNA or siLNA seqeunce
<220>
   <221> Beta-D-oxy LNA
   <222> (1) .. (1)
<220>
   <221> P=O backbone
   <222> (1)..(211)
<220>
   <221> RNA
   <222> (2)..(7)
<220>
   <221> Beta-D-oxy LNA
   <222> (8)..(8)
<220>
   <221> -RNA
   <222> (9)..(9)
<220>
   <221> Beta-D-oxy LNA
   <222> (10) .. (10)
<220>
   <221> RNA
   <222> (11)..(19)
<220>
   <221> Beta-D-oxy LNA
   <222> (20)..(21)
<400> 5
   ccuacugcag ggugaagaat t 21
<210> 6
   <211> 21
   <212> DNA
   <213> Artificial
<220>
   <223> siRNA/siLNA sequence
<220>
   <221> Beta-D-oxy LNA
   <222> (1)..(1)
<220>
   <221> P=S backbone
   <222> (1)..(21)
<220>
   <221> RNA
   <222> (2)..(7)
<220>
   <221> Beta-D-oxy LNA
   <222> (8)..(8)
<220>
   <221> RNA
   <222> (9)..(9)
<220>
   <221> Beta-D-oxy LNA
   <222> (10)..(10)
<220>
   <221> RNA
   <222> (11)..(19)
<220>
   <221> Beta-D-oxy LNA
   <222> (20)..(21)
<400> 6
   ccuacugcag ggugaagaat t 21
<210> 7
   <211> 21
   <212> DNA
   <213> Artificial
<220>
   <223> siRNA/siLNA sequence
<220>
   <221> Beta-D-oxy LNA
   <222> (1)..(1)
<220>
   <221> P=O backbone
   <222> (1)..(21)
<220>
   <221> RNA
   <222> (2).. (7)
<220>
   <221> Beta-D-oxy LNA
   <222> (8) .. (8)
<220>
   <221> RNA
   <222> (9)..(9)
<220>
   <221> Beta-D-oxy LNA
   <222> (10)..(10)
<220>
   <221> RNA
   <222> (11)..(13)
<220>
   <221> Beta-D-oxy LNA
   <222> (14)..(14)
<220>
   <221> RNA
   <222> (15)..(19)
<400> 7
   ccuacugcag ggugaagaat t 21
<210> 8
   <211> 21
   <212> DNA
   <213> Artificial
<220>
   <223> siRNA/siLNA sequence
<220>
   <221> Beta-D-oxy LNA
   <222> (1).. (1)
<220>
   <221> P=S backbone
   <222> (1)..(21)
<220>
   <221> RNA
   <222> (2)..(7)
<220>
   <221> Beta-D-oxy LNA
   <222> (8)..(8)
<220>
   <221> RNA
   <222> (9)..(9)
<220>
   <221> Beta-D-oxy LNA
   <222> (10)..(10)
<220>
   <221> RNA
   <222> (11)..(13)
<220>
   <221> Beta-D-oxy LNA
   <222> (14)..(14)
<220>
   <221> RNA
   <222> (15)..(19)
<220>
   <221> Beta-D-oxy LNA
   <222> (20)..(21)
<900> 8
   ccuacugcag ggugaagaat t 21
<210> 9
   <211> 21
   <212> DNA
   <213> Artificial
<220>
   <223> siRNA/siLNA sequence
<220>
   <221> RNA
   <222> (1) (19)
<220>
   <221> P=O backbone
   <222> (1)..(21)
<220>
   <221> deoxythymidine
   <222> (20)..(21)
<400> 9
   ccuacugcag ggugaagaat t 21
<210> 10
   <211> 21
   <212> DNA
   <213> Artificial
<220>
   <223> siRNA/siLNA sequence
<220>
   <221> RNA
   <222> (1)..(19)
<220>
   <221> P=S backbone
   <222> (1)..(21)
<220>
   <221> deoxythymidine
   <222> (20)..(21)
<400> 10
   ccuacugcag ggugaagaat t 21
<210> 11
   <211> 21
   <212> DNA
   <213> Artificial
<220>
   <223> siRNA/siLNA sequence
<220>
   <221> Beta-D-oxy LNA
   <222> (1)..(1)
<220>
   <221> P=S backbone
   <222> (1)..(21)
<220>
   <221> RNA
   <222> (2)..(7)
<220>
   <221> thiolated diesesterbond
   <222> (2)..(7)
<220>
   <221> Beta-D-oxy LNA
   <222> (8)..(8)
<220>
   <221> RNA
   <222> (9) .. (9)
<220>
   <221> thiolated diesesterbond
   <222> (9) .. (9)
<220>
   <221> Beta-D-oxy LNA
   <222> (10)..(10)
<220>
   <221> RNA
   <222> (11)..(19)
<220>
   <221> thiolated diesesterbond
   <222> (11)..(19)
<220>
   <221> Beta-D-oxy LNA
   <222> (20)..(21)
<400> 11
   ccuacugcag ggugaagaat t 21
<210> 12
   <211> 21
   <212> DNA
   <213> Artificial
<220>
   <223> siRNA/siLNA sequence
<220>
   <221> Beta-D-oxy LNA
   <222> (1)..(1)
<220>
   <221> P=S backbone
   <222> (1)..(21)
<220>
   <221> RNA
   <222> (2)..(7)
<220>
   <221> thiolated diesesterbond
   <222> (2)..(7)
<220>
   <221> Beta-D-oxy LNA
   <222> (8)..(8)
<220>
   <221> RNA
   <222> (9)..(9)
<220>
   <221> thiolated diesesterbond
   <222> (9) .. (19)
<220>
   <221> Beta-D-oxy LNA
   <222> (10)..(10)
<220>
   <221> RNA
   <222> (11)..(19)
<220>
   <221> Beta-D-oxy LNA
   <222> (20)..(21)
<400> 12
   ccuacugcag ggugaagaat t 21
<210> 13
   <211> 22
   <212> DNA
   <213> Artificial
<220>
   <223> siRNA/siLNA sequence
<220>
   <221> Beta-D-oxy LNA
   <222> (1)..(1)
<220>
   <221> P=O backbone
   <222> (1)..(21)
<220>
   <221> RNA
   <222> (2)..(7)
<220>
   <221> Beta-D-oxy LNA
   <222> (8)..(8)
<220>
   <221> RNA
   <222> (9)..(9)
<220>
   <221> Beta-D-oxy LNA
   <222> (10)..(10)
<220>
   <221> thiolated diesesterbond
   <222> (10)..(10)
<220>
   <221> RNA
   <222> (11)..(19)
<220>
   <221> Beta-D-oxy LNA
   <222> (20)..(21)
<400> 13
   ccuacugcag sggugaagaa tt 22
<210> 14
   <211> 21
   <212> DNA
   <213> Artificial
<220>
   <223> siRNA/siLNA sequence
<220>
   <221> RNA
   <222> (1)..(19)
<220>
   <221> P=O backbone
   <222> (1)..(21)
<220>
   <221> Beta-D-oxy LNA
   <222> (20)..(21)
<400> 14
   uucuucaccc ugcaguaggt t 21
<210> 15
   <211> 21
   <212> DNA
   <213> Artificial
<220>
   <223> siRNA/siLNA sequence
<220>
   <221> RNA
   <222> (1)..(19)
<220>
   <221> P=S backbone
   <222> (1)..(21)
<220>
   <221> Beta-D-oxy LNA
   <222> (20)..(21)
<400> 15
   uucuucaccc ugcaguaggt t 21
<210> 16
   <211> 21
   <212> DNA
   <213> Artificial
<220>
   <223> siRMA/siLNA sequence
<220>
   <221> RNA
   <222> (1)..(21)
<220>
   <221> P=S backbone
   <222> (1)..(21)
<220>
   <221> deoxythymidine
   <222> (20)..(21)
<400> 16
   uucuucaccc ugcaguaggt t 21
<210> 17
   <211> 21
   <212> DNA
   <213> Artificial
<220>
   <223> siRNA/siLNA sequence
<220>
   <221> RNA
   <222> (1)..(21)
<220>
   <221> P=O backbone
   <222> (1)..(21)
<220>
   <221> deoxythymidine
   <222> (20)..(21)
<400> 17
   uucuucaccc ugcaguaggt t 21